# EUROPÄISCHE PATENTANMELDUNG

(11) **EP 0 535 576 A1**
(43) Veröffentlichungstag der Anmeldung: **07.04.1993**
(21) Anmeldenummer: 92116577.5
(22) Anmeldetag: 28.09.1992
(51) Int. Cl.: C12N 15/87, A61K 47/48, C07K 15/28, C12P 21/08, C12N 15/34

(54) **Neue Konjugate zum Einführen von Nukleinsäure in höhere eukaryotische Zellen**

(30) Priorität: 30.09.1991 US 767787; 30.01.1992 US 827049; 07.04.1992 US 864758
(71) Anmelder: BOEHRINGER INGELHEIM INTERNATIONAL GmbH, D-55216 Ingelheim am Rhein (DE); GENENTECH, INC., South San Francisco California 94080 (US); UNIVERSITY OF NORTH CAROLINA AT CHAPEL HILL, Chapel Hill, N.C. 27599-7020 (US)
(72) Erfinder: Curiel, David, Chapel Hill, N.C. 27516 (US); Hu, Ping-Chuang, Chapel Hill, N.C. 27516 (US); Wagner, Ernst, A-2103 Langenzersdorf (AT); Birnstiel, Max L., A-110 Wien (AT); Cotten, Matt, A-1130 Wien (AT)
(74) Vertreter: Laudien, Dieter, Dr.

(57) **Zusammenfassung**

Konjugate, in denen ein Virus über einen Antikörper an eine Nukleinsäure-affine Substanz gebunden ist, für den Transport von Genkonstrukten in höhere eurkaryotische Zellen. Komplexe aus den Konjugaten und Nukleinsäure werden, gegebenenfalls unter Mitwirkung eines zelltypspezifischen Internalisierungsfaktors, in die Zelle internalisiert, wobei das Virus als Bestandteil des Komplexes die Internalisierung sowie die Freisetzung des Inhalts der Endosomen, in denen die Komplexe nach Eintritt in die Zelle lokalisiert sind, bewirkt. Pharmazeutische Zubereitungen, in denen die Nukleinsäure ein therapeutisch wirksames Genkonstrukt ist, insbesondere zur Verwendung für die Gentherapie, und Transfektionskits.

## Beschreibung

Die Erfindung bezieht sich auf das Einbringen von Nukleinsäuren in höhere eukaryotische Zellen.

Bedarf an einem effizienten System für das Einführen von Nukleinsäure in lebende Zellen besteht vor allem im Rahmen der Gentherapie. Dabei werden Gene in Zellen eingeschleust, um in vivo die Synthese therapeutisch wirksamer Genprodukte zu erzielen, z.B. um im Falle eines genetischen Defekts das fehlende Gen zu ersetzen. Die "klassische" Gentherapie beruht auf dem Prinzip, durch eine einmalige Behandlung eine dauernde Heilung zu erzielen. Daneben besteht jedoch Bedarf an Behandlungsmethoden, bei denen die therapeutisch wirksame DNA (oder auch mRNA) wie ein Medikament ("Gentherapeutikum") je nach Bedarf einmalig oder wiederholt verabreicht wird. Beispiele für genetisch bedingte Erkrankungen, bei denen die Gentherapie einen erfolgversprechenden Ansatz darstellt, sind Hämophilie, beta-Thalassämie und "Severe Combined Immune Deficiency" (SCID), ein Syndrom, das durch einen genetisch bedingten Mangel des Enzyms Adenosindeaminase hervorgerufen wird. Anwendungsmöglichkeiten bestehen weiters bei der Immunregulation, wobei durch Verabreichung funktioneller Nukleinsäure, die für ein sekretiertes Protein-Antigen oder für ein nichtsezerniertes Protein-Antigen kodiert, mittels einer Impfung eine humorale oder intrazelluläre Immunität erzielt wird. Weitere Beispiele für genetische Defekte, bei denen eine Verabreichung von Nukleinsäure, die für das defekte Gen kodiert, z.B. in individuell auf den Bedarf abgestimmter Form verabreicht werden kann, sind Muskeldystrophie (Dystrophin-Gen), Cystische Fibrose ("Cystic fibrosis transmembrane conductance regulator gene"), Hypercholesterolämie (LDL-Rezeptor-Gen). Gentherapeutische Behandlungsmethoden sind weiters potentiell dann von Bedeutung, wenn Hormone, Wachstumsfaktoren oder cytotoxisch oder immunmodulierend wirkende Proteine im Organismus synthetisiert werden sollen.

Die Gentherapie stellt auch einen erfolgversprechenden Ansatz für die Behandlung von Krebs dar, wobei sog. "Krebsvaccine" verabreicht werden. Um die Immunogenizität von Tumorzellen zu erhöhen, werden diese verändert, um sie entweder stärker antigenisch zu machen, oder um sie zu veranlassen, bestimmte immunmodulierende Substanzen zu erzeugen, z.B. Zytokine, die dann eine Immunantwort auslösen. Um dies zu bewirken, werden die Zellen mit DNA transfiziert, die für ein Zytokin, z.B. IL-2, IL-4, IFN-gamma, TNF-α, kodiert. Bisher wurde Gentransfer in autologe Tumorzellen hauptsächlich mittels retroviraler Vektoren durchgeführt.

Die Wirkungsweise von Antisense RNAs und -DNAs sowie von Ribozymen ermöglicht deren Anwendung als Therapeutika zur Blockierung der Expression bestimmter Gene (wie deregulierter Onkogene oder viraler Gene) in vivo. Es wurde bereits gezeigt, daß kurze Antisense-Oligonukleotide in Zellen importiert werden und dort ihre inhibierende Wirkung ausüben können (Zamecnik et al., 1986), wenngleich ihre intrazelluläre Konzentration, u.a. wegen ihrer beschränkten Aufnahme durch die Zellmembran auf Grund der starken negativen Ladung der Nukleinsäuren, gering ist.

Für die Gentransformation von Säugetierzellen in vitro sind verschiedene Techniken bekannt, deren Anwendbarkeit in vivo jedoch beschränkt ist (dazu zählen das Einbringen von DNA mittels Liposomen, Elektroporation, Mikroinjektion, Zellfusion, DEAE-Dextran oder die Calciumphosphat-Präzipitationsmethode).

In jüngster Zeit wurden biologische Vektoren entwickelt, die den Transfer von Genen bewerkstelligen, indem sie sich der effizienten Eintrittsmechanismen ihrer Ausgangsviren bedienen. Diese Strategie wurde bei der Konstruktion rekombinanter retroviraler und adenoviraler Vektoren angewendet, um einen hoch wirksamen Gentransfer *in vitro* und *in vivo* zu erzielen (Berkner, 1988). Bei all ihrer Wirksamkeit sind diese Vektoren Beschränkungen, und zwar hinsichtlich der Größe und Konstruktion der zu transferierenden DNA, unterworfen. Außerdem bringen diese Mittel Sicherheitsrisken aufgrund des Co-Transfers von lebensfähigen viralen Gen-Elementen des Ursprungsvirus mit sich. So ist z.B. die Verwendung von Retroviren problematisch, weil sie, zumindest zu einem geringen Prozentsatz, die Gefahr von Nebenwirkungen wie Infektion mit dem Virus (durch Rekombination mit endogenen Viren oder Kontamination mit Helferviren und mögliche anschließende Mutation zur pathogenen Form) oder Entstehung von Krebs in sich birgt. Außerdem ist die stabile Transformation der somatischen Zellen des Patienten, wie sie mit Hilfe von Retroviren erzielt wird, nicht in jedem Fall wünschenswert, weil dadurch die Behandlung, z.B. bei Auftreten von Nebeneffekten, nur mehr schwierig rückgängig gemacht werden kann.

Um diese Beschränkungen zu umgehen, wurden alternative Strategien für den Gentransfer entwickelt, denen Mechanismen zugrundeliegen, deren sich die Zelle für den Transport von Makromolekülen bedient. Ein Beispiel dafür ist der Import von Genen in die Zelle über den äußerst leistungsfähigen Weg der Rezeptor-vermittelten Endozytose (Wu und Wu, 1987, Wagner et al., 1990, und EP-A1 0388 758). Dieser Ansatz bedient sich bifunktioneller molekularer Konjugate, die eine DNA-Bindungsdomäne und eine Domäne mit Spezifität für einen Zelloberflächen-Rezeptor aufweisen (Wu und Wu, 1987, Wagner et al., 1990). Wenn die Erkennungsdomäne (im folgenden als "Internalisierungsfaktor" bezeichnet) vom Zelloberflächen-Rezeptor erkannt wird, wird das Konjugat über den Weg der Rezeptor-vermittelten Endozytose internalisiert, wobei die an das Konjugat gebundene DNA mittransportiert wird. Mit Hilfe dieser Methode konnten Gentransfer-Raten erzielt werden, die den herkömmlichen Methoden zumindest ebenbürtig waren (Zenke et al., 1990).

Während dieses Vektorsystem große Mengen von DNA in Zellen transportieren kann, die den geeigneten Zelloberflächenrezeptor besitzen, steht die entsprechende Gen-Expression sehr oft nicht im Einklang mit der Transportkapazität (Cotten et al., 1990). Es wurde u.a. angenommen, daß die Ursache für dieses Phänomen darin gelegen ist, daß die über Rezeptorvermittelte Endozytose in die Zelle beförderte DNA in Lysosomen landet, wo sie einem Abbau unterliegt (Zenke et al., 1990, Cotten et al., 1990). Daher stellt die Tatsache, daß die in Lysosomen internalisierte DNA über keinen spezifischen Mechanismus verfügt, aus dem intrazellulären Vesikelsystem auszutreten, eine diesem Transportsystem immanente Beschränkung dar.

Der vorliegenden Erfindung lag die Aufgabe zugrunde, diese Beschränkungen zu reduzieren bzw. zu beseitigen.

Eine Vielzahl von Viren bewerkstelligen ihr Eindringen in den eukaryotischen Wirt über Mechanismen, die im Prinzip denen der Rezeptor-vermittelten Endozytose entsprechen. Eine Virusinfektion auf Basis dieses Mechanismus beginnt im allgemeinen mit der Bindung von Viruspartikeln an Rezeptoren auf der Zellmembran. Im Anschluß daran erfolgt die Internalisierung des Virus in die Zelle. Dieser Internalisierungsvorgang folgt einer gemeinsamen Route, entsprechend dem Eintritt von physiologischen Liganden oder von Makromolekülen in die Zelle: Die Rezeptoren auf der Zelloberfläche ordnen sich zunächst in Gruppen an, um ein sog. "coated pit" zu bilden, daraufhin stülpt sich die Membran nach innen und bildet ein von einer Hülle umgebenes Vesikel. Nachdem sich dieses Vesikel seiner Clathrin-Hülle entledigt hat, erfolgt in seinem Inneren mit Hilfe einer in der Membran lokalisierten Protonenpumpe eine Ansäuerung. Dadurch wird die Freisetzung des Virus aus dem Endosom hervorgerufen. In Abhängigkeit davon, ob das Virus eine Lipidhülle aufweist oder nicht, wurden zwei Arten der Freisetzung des Virus aus dem Endosom in Betracht gezogen: Im Fall von sog. "nackten" Viren (z.B. Adenovirus, Poliovirus, Rhinovirus) wurde vorgeschlagen, daß der niedrige pH-Wert Konformationsänderungen in Virusproteinen hervorruft. Dadurch werden hydrophobe Domänen freigelegt, die bei physiologischem pH-Wert nicht zugänglich sind. Diese Domänen erlangen dadurch die Fähigkeit, mit der Endosomenmembran in Wechselwirkung zu treten und damit die Freisetzung des Virusgenoms aus dem Endosom ins Zytoplasma zu bewirken. Von Viren mit Hülle (z.B. Vesicular Stomatitis Virus, Semliki Forest Virus, Influenza Virus) wird vermutet, daß der niedrige pH-Wert die Struktur oder Konformation einiger Virusproteine modifiziert, wodurch die Fusion der Virusmembran mit der Endosomenmembran gefördert wird. Viren, die mit Hilfe dieses Mechanismus in die Zelle eindringen, weisen bestimmte molekulare Besonderheiten auf, die es ihnen ermöglichen, die Endosomenmembran aufzubrechen, um sich Eintritt ins Zytoplasma zu verschaffen.

Andere Viren, z.B. die eine Hülle aufweisenden Viren Sendai, HIV und einige Moloney Leukämie-Virusstämme, oder die keine Hülle aufweisenden Viren SV40 und Polyoma benötigen für ihr Eindringen in die Zelle kein niedriges pH-Milieu, können entweder direkt an der Zelloberfläche eine Fusion mit der Membran herbeiführen (Sendai Virus, möglicherweise HIV) oder aber sie vermögen Mechanismen auszulösen, um die Zellmembran aufzubrechen oder sie zu passieren. Es wird angenommen, daß auch die pH-Wert-unabhängigen Viren den Endozytose-Weg benutzen können (McClure et al., 1990).

In Experimenten, die der vorliegenden Erfindung vorausgegangen sind, wurde festgestellt, daß der Gentransfer mittels Nukleinsäure-Komplexen, in denen die Nukleinsäure mit Polykationen, gegebenenfalls gekoppelt an einen Internalisierungsfaktor, komplexiert ist, z.B. mit Transferrin-Polylysin-Konjugaten, durch die Behandlung mit Adenoviren, bestimmten Retroviren oder mit Virusfragmenten Viren stark erhöht wird. Dieser Effekt wurde durch Ausnutzung des Phänomens erzielt, daß diese Viren in die Zellen über Endozytose-Vorgänge aufgenommen werden und über einen spezifischen Mechanismus verfügen, aus dem Vesikelsystem zu entkommen, indem sie, z.B. im Fall der Adenoviren, die Endosomen aufbrechen (Pastan et al., 1986).

Ausgehend von diesen Beobachtungen wurde die gestellte Aufgabe durch die Entwicklung eines Biokonjugats gelöst, das das Virus als integralen Bestandteil seines funktionellen Konstrukts enthält.

Die vorliegende Erfindung betrifft somit ein Konjugat, das die Fähigkeit besitzt, mit Nukleinsäure Komplexe zu bilden und das aus einem Internalisierungsfaktor und einer Nukleinsäure-affinen Substanz besteht, zum Einführen von Nukleinsäuren in höhere eukaryotische Zellen. Das Konjugat ist dadurch gekennzeichnet, daß der Internalisierungsfaktor ein Virus ist, das an die Nukleinsäure-bindende Substanz über einen Antikörper derart gebunden ist, daß es die Fähigkeit aufweist, als Bestandteil des Konjugat/Nukleinsäure-Komplexes in die Zelle einzudringen und den Inhalt der Endosomen, in denen der Komplex nach Eintritt in die Zelle lokalisiert ist, ins Zytoplasma freizusetzen.

Die Erfindung betrifft in einem weiteren Aspekt Komplexe, in denen die erfindungsgemäßen Konjugate mit Nukleinsäure komplexiert sind.
Die Fähigkeit des Virus, in die Zelle einzudringen und den Inhalt der Endosomen, in denen der Konjugat/Nukleinsäure-Komplex lokalisiert ist, ins Zytoplasma freizusetzen, wird im folgenden als "Aufnahmefunktion" bezeichnet.

Die erfindungsgemäßen Konjugate vereinigen die Vorteile von auf Internalisierungsfaktor-Konjugaten basierenden Vektorensystemen mit den Vorteilen, die die Viren in diese Systeme einbringen.
Gegenüber dem Gentransfer über Rezeptor-vermittelte Endozytose weisen die erfindungsgemäßen Virus-Polykation-DNA-Komplexe den Vorteil auf, daß sie die grundsätzliche Beschränkung, die den bekannten molekularen Konjugat-Systemen inhärent ist, umgehen, indem sie im Gegensatz zu den vorbekannten Konjugaten über einen spezifischen Mechanismus verfügen, der ihre Freisetzung aus dem Zellvesikel-System ermöglicht.

Gegenüber biologischen Vektoren stellt das erfindungsgemäße Vektorsystem eine grundsätzliche konzeptuelle Abkehr von den rekombinanten viralen Vektoren dar, indem die zu transportierende Fremd-DNA an der Außenseite des Virions getragen wird. Dadurch können mit Hilfe der erfindungsgemäßen Konjugate auch sehr große Genkonstrukte in die Zelle transportiert werden, wobei hinsichtlich der Sequenz keinerlei Beschränkungen bestehen.

Geeignete Viren sind einerseits solche, die die Fähigkeit besitzen, über Rezeptor-vermittelte Endozytose in die Zelle einzudringen und ihre Freisetzung - und damit die Freisetzung der Nukleinsäure - aus dem Endosom ins Zytoplasma herbeizuführen. (Die Eignung von Viren im Rahmen der vorliegenden Erfindung ist weiters dadurch definiert, daß sie diese Fähigkeit auch als Bestandteil der Nukleinsäure-Komplexe beibehalten.) Ohne auf diese Theorie festgelegt sein zu wollen, könnte dieser Mechanismus den in die Zelle importierten Nukleinsäure-Komplexen insofern zugute kommen, als aufgrund der Fähigkeit des Virus, den Inhalt der Endosomen freizusetzen, die Fusion zwischen Endosomen und Lysosomen und damit der in diesen Zellorganellen normalerweise stattfindene enzymatische Abbau vermieden wird.

Die höheren eukaryotischen Zellen sind dem Fachmann geläufig; Hefen werden nicht dazugezählt (Watson et al., 1987). Beispiele höherer eukaryotischer Zellen, die von Adenoviren infiziert werden können, wurden von Fields und Knipe, 1990, beschrieben.

Zu Viren, deren am Beginn der Infektion ablaufende Aufnahmefunktion über Rezeptor-vermittelte Endozytose erfolgt und die aufgrund dieser Eigenschaft als Bestandteil der erfindungsgemäßen Konjugate geeignet sind, zählen einerseits Viren ohne Lipid-Hülle wie Adenovirus, Poliovirus, Rhinovirus, andererseits die Hüllenviren Vesicular Stomatitis Virus, Semliki Forest Virus, Influenza-Virus; geeignet sind außerdem pH-Wertabhängige Stämme von Moloney-Virus. Zu besonders bevorzugten Viren für die Anwendung der vorliegenden Erfindung zählen Adenovirus, Untergruppe C, Typ 5, Semliki Forest Virus, Vesicular Stomatitis Virus, Poliovirus, Rhinoviren und Moloney Leukämie-Virusstämme. Die Verwendung von RNA-Viren, die keine reverse Transkriptase haben, für die vorliegende Erfindung hat den Vorteil, daß eine Transfektion in Gegenwart eines solchen Virus nicht die Bildung von viraler DNA in der Zelle zur Folge hat.

Ein wesentlicher Vorteil, der sich aus der vorliegenden Erfindung ergibt, ist, daß die zu transferierende DNA nicht, wie im Fall der rekombinanten viralen Vektoren (siehe Berkner, 1988; Eglitis und Anderson, 1988), in das Genom des Ausgangsvirus integriert ist. Daher bietet die vorliegende Erfindung hinsichtlich der Auswahl der zu exprimierenden Fremdgensequenz eine wesentlich größere Flexibilität, weil die Transkription nicht von Promotoren im Ausgangsvirus abhängt. Außerdem erlaubt diese Strategie eine beträchtliche Erhöhung der Größe der DNA, die transferiert werden kann, da die Beschränkungen, die das Virus hinsichtlich der Verpackung auferlegt, die Menge an DNA, die an der Außenseite getragen werden kann, nicht limitiert. Über diese praktischen und unmittelbaren Vorteile hinaus, leiten sich wesentliche mögliche Sicherheitsaspekte von der Konzeption des Vektors ab. Konventionelle rekombinante virale Vektoren bedingen einen zwangsläufigen Mittransport von Genomelementen des Ausgangsvirus, von dem sich mögliche Sicherheitsrisken ableiten (Ledlex, 1989; Anderson, 1984). Da die erfindungsgemäßen Konjugate selektiv die virale Aufnahmefunktion ausnützen, ist das Virusgenom kein wesentliches Merkmal. Dieses Konzept erlaubt die Möglichkeit der Modifikation der vorliegenden Erfindung mit einem funktionell und/oder strukturell inaktiviertem viralem Genom, um die Sicherheitsrisken zu minimieren, die sich aus dem Transfer von lebensfähigen Genen aus dem Ausgangsvirus ergeben.

Im Rahmen der vorliegenden Erfindung werden unter dem Begriff Viren - vorausgesetzt, daß sie sie die Aufnahmefunktion im Sinne obiger Definition aufweisen - außer den Wildtypen auch Mutanten verstanden, denen aufgrund einer oder mehrerer Mutationen Funktionen des Wildtyps, die von der Aufnahmefunktion verschieden sind, insbesondere die Replikationsfähigkeit, abhanden gekommen sind. Es können jedoch Mutanten, die ihre Aufnahmefunktion verloren haben, im Rahmen der vorliegenden Erfindung angewendet werden, sofern sie als Teil eines ternären Komplexes gemäß obiger Definition eingesetzt werden und das mutante Virus seine endosomolytische Aktivität nicht verloren hat.

Derartige Mutanten werden durch Mutationen in Virus-Protein-Regionen, die für Replikationsfunktionen verantwortlich und für die Aufnahmefunktion entbehrlich sind und die durch die Verpackungslinie komplementiert werden können, mittels herkömmlicher Mutageneseverfahren hergestellt. Dazu zählen, z.B. im Fall von Adenovirus, ts-Mutanten (temperatursensitive Mutanten), E1A- und E1B-Mutanten, Mutanten, die Mutationen in MLP-getriebenen Genen aufweisen (Berkner, 1988) und Mutanten, die Mutationen in Bereichen bestimmter Kapsidproteine aufweisen. Geeignet sind weiters Virusstämme, die entsprechende natürliche Mutationen aufweisen. Die Replikationsfähigkeit der Viren kann z.B. mit Hilfe literaturbekannter Plaque-Assays untersucht werden, bei denen Zellkulturen mit Suspensionen unterschiedlicher Viruskonzentration beschichtet werden und die Zahl der lysierten Zellen, die an Hand von Plaques sichtbar ist, festgestellt wird (Dulbecco, 1980).

Für die Anwendung im Rahmen der vorliegenden Erfindung geeignet sind außerdem sog. defekte Viren, das sind Viren, denen in einem oder mehreren Genen die für die autonome Virusreplikation erforderliche Funktion fehlt, für die sie Helferviren benötigen. Vertreter dieser Gruppe sind DI-Partikel ("defective interfering particles"), die sich vom infektiösen Standard-Virus ableiten, dieselben Strukturproteine wie das Standardvirus besitzen, Mutationen aufweisen und das Standardvirus als Helfervirus für ihre Replikation benötigen (Huang, 1987, Holland, 1990). Vertreter dieser Gruppe sind außerdem die Satellitenviren (Holland, 1990). Eine andere Gruppe ist die Klasse der Parvoviren, die als "adeno-associated virus" (Berns, 1990) bezeichnet werden.

Da die Aufnahmezyklen vieler Viren in die Zelle noch nicht zur Gänze aufgeklärt sind, ist anzunehmen, daß es noch andere Viren gibt, die die endosomolytische Aktivität aufweisen, die für ihre Eignung zur Anwendung in der vorliegenden Erfindung erforderlich ist.

Weiters im Rahmen der vorliegenden Erfindung geeignet sind attenuierte Lebendvaccine (Ginsberg, 1980) oder Impfstämme.

Weiters fallen unter den Begriff Viren im Rahmen der vorliegenden Erfindung inaktivierte Viren, z.B. durch chemische Behandlung, wie Formaldehydbehandlung, durch UV-Bestrahlung, durch chemische Behandlung, kombiniert mit UV-Bestrahlung, z.B. Psoralen/UV-Behandlung, durch gamma-Strahlung oder durch Neutronenbeschuß inaktivierte Viren, weiters Teile von Viren, z.B. der von Nukleinsäure befreite Proteinanteil (das leere Viruskapsid), sofern sie die Aufnahmefunktionen des intakten Virus besitzen.

Inaktivierte Viren, wie sie z.B. auch für Vaccine verwendet werden, können mittels literaturbekannter Standardmethoden (Davis und Dulbecco, 1980, Hearst und Thiry, 1977) hergestellt und daraufhin untersucht werden, ob sie sich als Komponente der erfindungsgemäßen Konjugate eignen.

Gegebenenfalls ist das Virus ein chimäres Virus, das in einer Region, die für die Aufnahmefunktion nicht wesentlich ist, ein Fremdepitop aufweist. Jedoch können chimäre Viren, wenn sie auch ihre Aufnahmefunktion verloren haben, im Rahmen von Kombinationskomplexen eingesetzt werden, solange das Virus seine endosomolytischen Eigenschaften nicht verloren hat.

Um ein Virus, ein inaktiviertes Virus oder eine Viruskomponente für die im einzelnen durchzuführende Transfektion auszuwählen, wird z.B. so vorgegangen, daß das Virus zunächst in Vorversuchen daraufhin untersucht wird, ob es einen Effekt bei der Aufnahme von Nukleinsäure/Polykation-Komplexen in die Zielzelle zeigt. Weiters können seine Aufnahmefunktionen getestet werden, indem es bei der Transfektion mit Biokonjugaten, z.B. Transferrin-Polykation-Konjugaten bzw. einem anderen Biokonjugat mit Spezifität für die zu transfizierende Zielzelle, angewendet und seine Fähigkeit zur Steigerung der Gentransferkapazität durch Messung der Expression eines Reportergens überprüft wird.

Im Falle der Verwendung intakter Viren wird, zweckmäßigerweise parallel zu den Vorversuchen, in denen das Virus auf seine Eignung für die vorgesehene Transfektion untersucht wird, geprüft, ob das Virus replikationsfähig ist. Die Untersuchung auf Replikationsfähigkeit wird im Fall von zythopathischen Viren oder im Fall von Viren, die das Wachstum der Wirtszellen merklich beeinträchtigen, mit Hilfe von Plaque-Assays durchgeführt (vgl. oben). Bei anderen Viren werden Nachweismethoden speziell für das jeweilige Virus angewendet, z.B. der Hämagglutionationstest oder chemisch-physikalische Methoden (elektronenmikroskopisch) .

Es werden im Rahmen der vorliegenden Erfindung solche Viren bevorzugt, die in hohem Titer herstellbar sind, die stabil sind, die als Wildtyp geringe Pathogenität aufweisen und bei denen eine gezielte Ausschaltung der Replikationsfunktionen möglich ist, insbesondere Adenoviren. Wenn eine bestimmte Zellpopulation transfiziert werden soll, sind Viren bevorzugt, die diese Zellpopulation spezifisch infizieren. Für den Fall, daß von der Transfektion verschiedene Zelltypen erfaßt werden sollen, werden Viren eingesetzt, die für weiten Bereich von Zelltypen infektiös sind.

In jedem Fall kommen für die therapeutische Anwendung der vorliegenden Erfindung in vivo nur solche Viren bzw. Viruskomponenten in Betracht, bei denen Sicherheitsrisken, insbesondere bzgl. der Replikation des Virus in der Zelle sowie der Rekombination von Virus-DNA mit Wirts-DNA, weitestgehend minimiert sind.

In Vorversuchen zur vorliegenden Erfindung wurden Adenovirus-Präparationen mittels einer herkömmlichen UV-Sterilisationslampe oder mit Formaldehyd inaktiviert und überrraschend festgestellt, daß das Ausmaß der Inaktivierung der Viren wesentlich größer war als die Abnahme des Gentransfer-Effekts. Dies ist ein deutlicher Hinweis darauf, daß Mechanismen, die beim aktiven Virus mit dem normalen Infektionsmechansimus in Zusammenhang stehen, zerstört werden können, ohne daß der für den Gentransfer wesentliche Effekt eliminiert wurde.

Im Rahmen der vorliegenden Erfindung geeignete Nukleinsäure-affine Substanzen sind z.B. homologe Polykationen wie Polylysin, Polyarginin, Polyornithin oder heterologe Polykationen mit zwei oder mehr unterschiedlichen positiv geladenen Aminosäuren, wobei diese Polykationen verschiedene Kettenlänge aufweisen können, weiters nichtpeptidische synthetische Polykationen wie Polyethylenimin. Geeignete Nukleinsäure-affine Substanzen sind weiters natürliche DNA bindende Proteine polykationischen Charakters wie Histone oder Protamine bzw. Analoge oder Fragmente davon.

Die Empfänglichkeit einer gegebenen Zellinie für die Transformation durch ein Virus, das den Eintritt von Konjugaten in die Zelle erleichtert bzw. einen Liganden für diesen Zelltyp darstellt, hängt vom Vorhandensein und der Anzahl von Oberflächenrezeptoren für das Virus auf der Zielzelle ab. Methoden zur Bestimmung der Zahl von Adenovirusrezeptoren auf der Zelloberfläche werden für HeLa- und KB-Zellen von Svensson, 1985, und Defer, 1990, beschrieben. Es wird angenommen, daß der Adenovirusrezeptor ziemlich ubiquitär exprimiert wird.

Daher sind viele Zellinien mit einem Vektorsystem, das ein Adenovirus oder einen Teil davon enthält, transformierbar. Einige höhere eukaryotische Zellen haben jedoch wenige oder keine virale Rezeptoren. Wenn solche Zellen transformiert werden sollen, kann es notwendig sein, ein zweites Konjugat eines Internalisierungsfaktors einzusetzen, der an eine Nukleinsäure-affine Substanz gebunden ist, wobei der Internalisierungsfaktor spezifisch für einen Oberflächenrezeptor der höheren eukaryotischen Zelle ist und wobei das Viruskonjugat und das Internalisierungsfaktorkonjugat mit der Nukleinsäure komplexiert sind. Solche Komplexe können mit Erfolg eingesetzt werden, um die Transformation höherer eukaryotischer Zellen, wie z.B. epitheliale Atemwegszellen, die eine relativ geringe Zelloberflächenpopulation von Adenovirusrezeptoren haben (z.B. die Zellinie HBE1), zu verstärken.

In einer bevorzugten Ausführungsform der Erfindung enthalten die Komplexe daher zusätzlich zum Viruskonjugat ein weiteres Konjugat, in dem eine Nukleinsäure-affine Substanz, im Falle im allgemeinen dieselbe wie die des Konjugats, mit einem Internalisierungsfaktor mit Affinität für die Zielzelle gekoppelt ist. Diese Ausführungsform der vorliegenden Erfindung wird vor allem dann angewendet, wenn die Zielzelle keine oder nur wenige Rezeptoren für das Virus aufweist. In Gegenwart eines weiteren Internalisierungsfaktor-Verbindungsfaktor-Konjugats profitieren diese endosomolytischen Konjugate von der Internalisierungsfähigkeit des zweiten Konjugats, indem sie gemeinsam mit diesem an die Nukleinsäure komplexiert werden und als Bestandteil des so entstandenen Komplexes, im folgenden als "Kombinations-Komplex" oder "ternärer Komplex" bezeichnet, in die Zelle aufgenommen werden.

Im einzelnen kann in Vorversuchen bestimmt werden, ob die Anwendung eines (weiteren) Internalisierungsfaktors die Aufnahme von Nukleinsäure-Komplexen ermöglicht oder verbessert. Solche Test umfassen Parallel-Transfektionen mit Nukleinsäure-Komplexen einmal ohne zusätzlichen Internalisierungsfaktor, d.h. mit Komplexen, bestehend aus Nukleinsäure und Viruskonjugat, und einmal mit Komplexen, in denen die Nukleinsäure mit einem weiteren Konjugat, enthaltend einen Internalisierungsfaktor, für den die Zielzellen einen Rezeptor haben, komplexiert ist. Wenn ein zusätzlicher Internalisierungsfaktor verwendet wird, wird dieser vor allem durch die Zielzellen definiert, z.B. durch bestimmte Zelloberflächenantigene oder Rezeptoren, die für einen bestimmten Zelltyp spezifisch sind und daher den gezielten Transport der Nukleinsäure in diesen Zelltyp ermöglichen.

Unter Internalisierungsfaktor sind im Rahmen der vorliegenden Erfindung Liganden oder Fragmente davon zu verstehen, die nach Bindung an die Zelle über Endozytose, vorzugsweise Rezeptor-vermittelte Endozytose, internalisiert werden, oder Faktoren, deren Bindung/Internalisierung über Fusion mit Zellmembranelementen erfolgt.
Zu geeigneten Internalisierungsfaktoren zählen die Liganden Transferrin (Klausner et al., 1983), Conalbumin (Sennett et al., 1981), Asialoglykoproteine (wie Asialotransferrin, Asialorosomucoid oder Asialofetuin), (Ashwell et al., 1982), Lectine (Goldstein et al., 1980 und Shardon, 1987), bzw. Substanzen, die Galaktose enthalten und über den Asialoglykoproteinrezeptor internalisiert werden; mannosylierte Glykoproteine (Stahl et al., 1987), lysosomale Enzyme (Sly et al., 1982), LDL (Goldstein et al., 1982), modifizierter LDL (Goldstein et al., 1979), Lipoproteine, die über Rezeptoren in die Zelle aufgenommen werden (apo B100/LDL); virale Proteine, wie das HIV-Protein gp120; Antikörper (Mellman et al., 1984, Kuhn et al., 1982, Abrahamson et al., 1982) bzw. Fragmente davon gegen Zelloberflächenantigene, z.B. anti-CD4, anti-CD7; Zytokine wie Interleukin-1 (Mizel et al., 1987), Interleukin-2 (Smith et al., 1985), TNF (Imamure et al., 1987), Interferone (Anderson et al., 1982); CSF ("Colony-stimulating Factor"), (Walker et al., 1987), Faktoren und Wachstumsfaktoren wie Insulin (Marshall, 1985), EGF ("Epitermal Growth Factor"), (Carpenter, 1984); PDGF ("Platelet-derived Growth Factor"), (Heldin et al., 1982), TGFβ (Transforming Growth Factor β"), (Massague et al., 1986), Nervenwachstumsfaktor (Hosang et al., 1987), Insulinähnlicher Wachstumsfaktor I ("Insulin-like Growth Factor"), (Schalch et al., 1986), LH, FSH (Ascoli et al., 1978), Wachstumshormon (Hizuka et al., 1981), Prolactin (Posner et al., 1982), Glucagon (Asada-Kubota et al., 1983), Thyroidhormone (Cheng et al., 1980), α-2-Makroglobulin-Protease (Kaplan et al., 1979), sowie "entwaffnete" Toxine. Weitere Beispiele sind Immunglobuline oder Fragmente davon als Liganden für den Fc-Rezeptor oder Anti-Immunglobulin-Antikörper, die an SIgs ("Surface Immunoglobulins") binden. Die Liganden können natürlichen oder synthetischen Ursprungs sein (siehe Trends Pharmacol. Sci., 1989, und die darin zitierten Referenzen).

Wesentlich für die Eignung solcher Internalizierungsfaktoren im Rahmen der vorliegenden Erfindung ist,
a) daß sie vom spezifischen Zelltyp, in den die Nukleinsäure eingebracht werden soll, internalisiert werden können und ihre Internalisierungsfähigkeit nicht oder nicht wesentlich beeinträchtigt wird, wenn sie mit dem Verbindungsfaktor konjugiert werden, und
b) daß sie im Rahmen dieser Eigenschaft in der Lage sind, Nukleinsäure auf dem von ihnen benutzten Weg in die Zelle "huckepack" mitzunehmen.

Ohne auf diese Theorie festgelegt sein zu wollen, werden die Kombinations-Komplexe von Zellen entweder durch Bindung an den für den Internalisierungsfaktor spezifischen Oberflächenrezeptor oder, im Falle der Verwendung eines Virus oder einer Viruskomponente, durch Bindung an den Virusrezeptor oder durch Bindung an beide Rezeptoren über den Weg der Rezeptorvermittelten Endozytose aufgenommen. Bei der Freisetzung der endosomolytische Mittel aus den Endosomen wird auch die in den Komplexen enthaltene DNA in das Zytoplasma freigesetzt und entkommt dadurch dem lysosomalen Abbau.

Die Gegenwart von Viren, Viruskomponenten oder nichtviralen endosomolytischen Mitteln als Bestandteile von endosomolytischen Konjugaten in den DNA-Komplexen hat folgende Vorteile:
1) Breitere Anwendbarkeit der Gentransfertechnik mit Nukleinsäure-Komplexen, weil das endosomolytische Mittel selbst, insbesondere für den Fall, daß ein Virus oder eine Viruskomponente eingesetzt wird, den Internalisierungsfaktor darstellen kann oder auch in Kombination mit einem weiteren Internalisierunsfaktor (z.B. Transferrin oder Asialofetuin etc.) an die DNA komplexiert werden kann. Dadurch ist es möglich, den positiven Effekt der Viren auch für Zellen auszunützen, die keinen Rezeptor für die jeweiligen Viren haben.
2) Verbesserung der Gentransfer Effizienz, da durch die Bindung der endosomolytischen Konjugate an die DNA eine gemeinsame Aufnahme in die Zellen gewährleistet ist. Durch die koordinierte Aufnahme und Freisetzung von Viren und DNA, ergibt sich weiters die Möglichkeit einer Reduktion der für einen effizienten Gentransfer benötigten Menge an DNA und Viren, was besonders für Anwendungen *in vivo* von essentieller Bedeutung ist.

In den Versuchen, die im Rahmen der vorliegenden Erfindung durchgeführt wurden, wurde Humantransferrin als zusätzlicher Internalisierungsfaktor verwendet, außerdem wurde die Leistungsfähigkeit der erfindungsgemäßen Konjugate anhand von Komplexen aus DNA und Polylysin-konjugiertem Virus, die kein zusätzliches Internalisierungsfaktor-Bindungsfaktor-Konjugat enthielten, demonstriert.

Die Bindung des Virus an die Nukleinsäure-affine Substanz wird durch kovalente Bindung der Nukleinsäureaffinen Substanz an einen Antikörper hergestellt. Bevorzugt wird ein Antikörper verwendet, der an ein Epitop in einer Virusprotein-Region bindet, die an der Aufnahmefunktion des Virus nicht beteiligt ist.

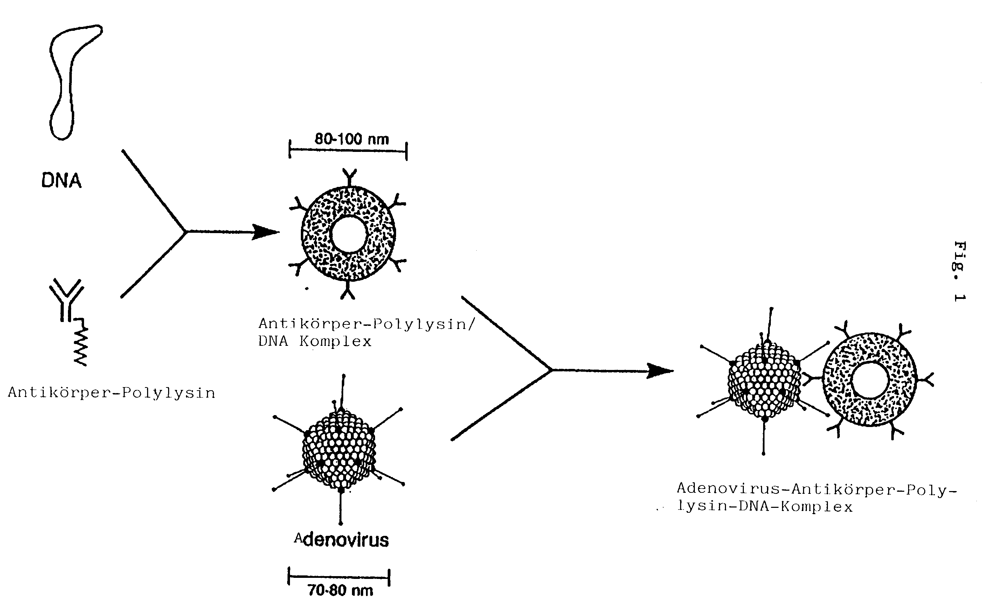

In den Versuchen, die im Rahmen der vorliegenden Erfindung durchgeführt wurden, wurde die Bindung zwischen einem Adenovirus und einem Polykation hergestellt, indem ein Antikörper mit Spezifität für das Adenovirus-Kapsid mit einem Polylysin-Molekül kovalent konjugiert wurde. Es ist bekannt, daß die Adenovirus-Faser- und Pentonproteine für die Bindung des Virus und seine Aufnahme in die Zelle wesentlich sind, während das Hauptkapsidprotein Hexon für diese Vorgänge eine geringere Rolle spielt. Es wurde daher ein Antikörper eingesetzt, der die Verbindung des Adenovirus mit Polylysin durch Erkennung eines Epitops auf dem Hexonprotein bewerkstelligt. Diese spezifische Verbindung wurde erzielt, indem einerseits ein chimäres Adenovirus verwendet wurde, das ein fremdes Epitop in der Oberflächenregion seines Hexonproteins aufweist. Andererseits wurde ein monoklonaler Antikörper eingesetzt, der spezifisch für das heterologe Epitop ist. (Diese Konstruktion ist schematisch in Fig. 1 dargestellt.) Dadurch wurde eine Verbindung des Adenovirus mit Polylysin erhalten, ohne die Kapsidproteine funktionell zu zerstören.

Die Verwendung eines speziellen Antikörpers zur Herstellung der Verbindung zwischen dem Virus und der Nukleinsäure-bindenden Substanz ist nicht kritisch. Voraussetzung für die Eignung eines bestimmten Antikörpers ist, daß er die Aufnahmefunktion des Virus nicht oder nur zum Teil neutralisiert.

Im Rahmen der vorliegenden Erfindung sind Antikörper gegen Epitope in Virusprotein-Regionen, die für die Aufnahmefunktion nicht essentiell sind, bevorzugt. Beispiele für derartige Virusregionen sind das bereits erwähnte Hexon-Protein des Adenovirus oder die Influenza-Neuraminidase.

Es sind jedoch auch Antikörper mit Spezifität für Virusproteine geeignet, die an der Aufnahmefunktion beteiligt sind, sofern durch Einhaltung eines geeigneten stöchiometrischen Verhältnisses gewährleistet ist, daß der Antikörper nur einen Teil der Zell-Bindungsregionen des Virus besetzt, sodaß noch ausreichend Domänen für die Bindung des Virus an die Zelle freibleiben. Andererseits können Antikörper, die die Aufnahmefunktion des Virus blockieren, verwendet werden, wenn der Komplex außerdem ein zweites Konjugat aus Internalisierungsfaktor und Nukleinsäure-bindender Substanz enthält, also ein Kombinationskomplex vorliegt.

Die für den spezifischen Anwendungsfall geeignete Antikörpermenge kann mit Hilfe von Titrationen bestimmt werden.

Bevorzugt sind monoklonale Antikörper, gegebenenfalls deren Fab'-Fragmente.

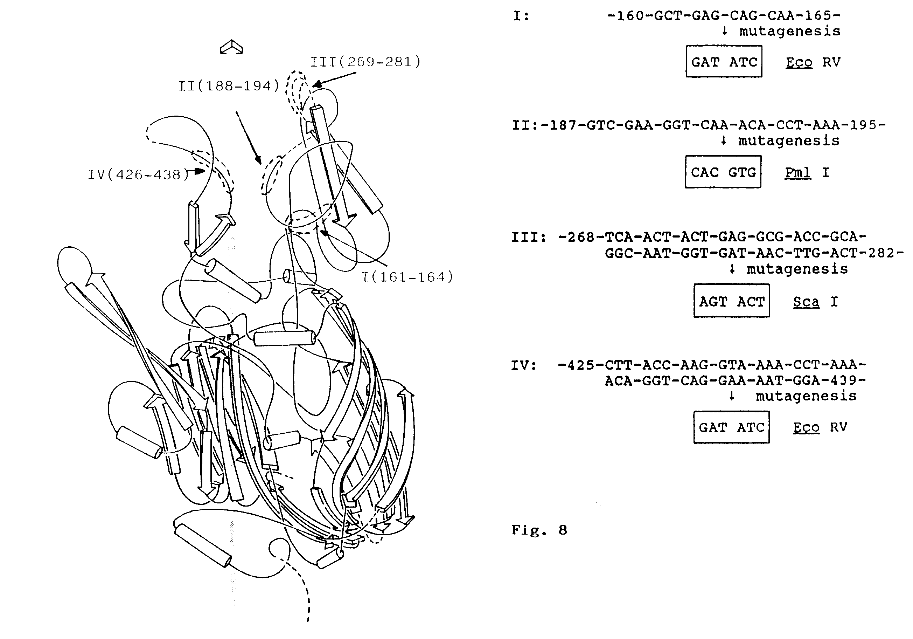

Falls das Virus ein chimäres Virus mit einem Fremdepitop ist, ist der Antikörper gegen dieses Epitop gerichtet. Vorzugsweise ist das Virus ein chimäres Adenovirus, in dem die für die Hexonregion kodierende Region modifiziert wurde, um eine Sequenz einzuschließen, die für ein heterologes Protein kodiert, gegen die ein Antikörper erzeugt werden kann. Das Hexonprotein setzt sich zusammen aus einer hochkonservierten Basisdomäne und drei weniger konservierten Schleifen, die an der Oberfläche des Virus stark exponiert sind (Roberts et al., 1986). In diesen Schleifen gibt es mehrere kurze Regionen, in denen die Ad2- und Ad5-Aminosäuresequenzen unterschiedlich sind, wobei Ad5 im Vergleich mit Ad2 sowohl Änderungen als auch Deletionen zeigt. Diese sind potentielle Stellen für die Einfügung der heterologen Gensequenzen, die für das heterologe Protein kodiert, das verwendet werden kann, um das Adenovirus immunologisch an die Nukleinsäure-affine Substanz zu binden. Vorzugsweise wird die heterologe Gensequenz in die Ad5-Gensequenz bei den Aminosäurepositionen 161-165, 188-194, 269-281 und 436-438 eingesetzt, auf die in Fig. 8 als Stellen I, II, III bzw. IV Bezug genommen wird. An jeder möglichen Stelle kann mittels "site-directed" Mutagenese eines Subklons des Ad5-Hexongens eine nur einmal vorkommende Restriktionsstelle geschaffen werden. Gleichzeitig können Nukleotide, die für nicht konservierte Aminosäuren kodieren, deletiert werden, wobei mehr Platz für die Insertion der heterologen Gensequenz bleibt. Aufgrund der kleinen Anzahl von Aminosäuren, die an den Stellen I, II, III und IV eingesetzt werden können (bis zu ca. 65 Aminosäuren), kodiert die heterologe Gensequenz im allgemeinen nur für die Aminosäuren, die dem Epitop entsprechen sowie einer Mindestanzahl von flankierenden Sequenzen (Die in der Ad5-Hexongensequenz mittels gerichteter Mutagenese geschaffenen möglichen Insertionsstellen sind in Fig. 8 dargestellt; für die dreidimensionale Skizze der Hexon-Untereinheit wurde die Darstellung von Roberts et al., 1986, adaptiert.)

Die Epitopspezifität eines speziellen monoklonalen Antikörpers für ein heterologes Protein kann mittels Peptid-Scanning bestimmt werden, siehe Geysen et al., 1984, 1985, 1986, 1987, und EP A 392 369, auf deren Offenbarungen Bezug genommen wird. Gemäß dieser Methode werden überlappende acht Aminosäuren lange Peptide des heterologen Proteins mittels Festphasensynthese hergestellt. Z.B. besteht Peptid 1 aus den Aminosäuren 1-8, Peptid 2 aus Aminosäuren 2-9, etc. Die Peptide bleiben nach der Synthese an den festen Träger gebunden. Die Überstände von Hybridomzellkulturen oder daraus gereinigte monoklonale Antikörper werden dann mittels ELISA auf Reaktivität mit den immobilisierten Peptiden getestet. Wenn einmal die epitope Region identifiziert ist, kann die Gensequenz, die für die eptitope Region kodiert, in jede der Restriktionsstellen der Regionen I, II, III oder IV eingefügt werden. Es gibt viele Beispiele von Proteinen und Antikörpern, die für das Protein spezifisch sind. Der Durchschnittsfachmann kann eine heterologe Protein/Antikörperkombination, die für die Anwendung im Rahmen der vorliegenden Erfindung geeignet ist, mit Hilfe von Versuchen, die nicht über Routineversuche hinausgehen, auswählen. Z.B. kann eine bekannte für ein Protein kodierende Sequenz, gegen die auch ein Antikörper bekannt ist bzw. aufgrund bekannter Methoden hergestellt werden kann, in die Hexonregion eines Adenovirus eingesetzt werden. Das resultierende chimäre Virus kann daraufhin auf seine immunologische Bindung, z.B. an einen markierten Antikörper, in einem Kompetitions-, ELISA- oder anderen Immunoassaytyp getestet werden. Derartige Immunoassaytechniken sind gut bekannt und können routinemäßig vom Durchschnittsfachmann durchgeführt werden.

Die Antikörper-Polykation-Konjugate können auf chemischem Weg in für die Kopplung von Peptiden an sich bekannter Weise hergestellt werden, wobei bevorzugt nach der von Wagner et al., 1990, und in der EP A1 388 758 beschriebenen Methode vorgegangen wird.

Sofern monoklonale Antikörper in der konstanten Region der schweren Kette geeignete Kohlenhydratseitenketten, insbesondere endständige Sialinsäuren, aufweisen, können die Konjugate durch Bindung des Polykations an die Kohlenhydratseitenkette hergestellt werden, wobei die von Wagner et al., 1991b, beschriebene Methode verwendet werden kann.

Die Erfindung betrifft in einem weiteren Aspekt Komplexe, die in höhere eukaryotische Zellen aufgenommen werden, enthaltend Nukleinsäure und ein Konjugat aus einem Internalisierungsfaktor und einer Nukleinsäure-affinen Substanz. Die Komplexe sind dadurch gekennzeichnet, daß der Internalisierungsfaktor ein Virus ist, das an die Nukleinsäure-affine Substanz über einen Antikörper derart gebunden ist, daß es die Fähigkeit besitzt, als Bestandteil des Konjugat/Nukleinsäure-Komplexes in die Zelle einzudringen und den Inhalt der Endosomen, in denen der Komplex nach Eintritt in die Zelle lokalisiert ist, ins Zytoplasma freizusetzen.

Hinsichtlich der qualitativen Zusammensetzung der Nukleinsäure-Komplexe wird im allgemeinen zunächst die in die Zelle zu importierende Nukleinsäure festgelegt. Die Nukleinsäure wird vor allem durch den in der Zelle zu erzielenden biologischen Effekt definiert, im Falle der Anwendung im Rahmen der Gentherapie durch das zur Expression zu bringende Gen bzw. den Genabschnitt, z.B. zwecks Substitution eines defekten Gens, oder durch die Zielsequenz eines zu inhibierenden Gens. Bei den in die Zelle zu transportierenden Nukleinsäuren kann es sich um DNAs oder RNAs handeln, wobei hinsichtlich der Nukleotidsequenz keine Beschränkungen bestehen.

Wenn die Erfindung auf Tumorzellen angewendet wird, um diese als Krebsvaccine einzusetzen, kodiert die in die DNA einzuführende Zelle vorzugsweise für eine immunmodulierende Substanz, z.B. ein Zytokin wie IL-2, IL-4, IFN-gamma, TNF-α. Von besonderem Nutzen können Kombinationen von DNAs sein, die für Zytokine kodieren, z.B. IL-2 und IFN-gamma. Ein weiteres Gen, das für das Einbringen in Tumorzellen nützlich ist, ist das "Multi Drug Restistance Gene" (mdr).

Es ist auch möglich, zwei oder mehr verschiedene Nukleinsäuresequenzen in die Zelle einzubringen, z.B. ein Plasmid, enthaltend cDNAs, kodierend für zwei verschiedene Proteine, unter der Kontrolle von geeigneten regulatorischen Sequenzen, oder zwei verschiedene Plasmidkonstrukte, enthaltend verschiedene cDNAs.

Zu therapeutisch wirksamen inhibierenden Nukleinsäuren, die zum Zweck der Inhibierung spezifischer Gensequenzen in die Zelle eingeführt werden, zählen Genkonstrukte, von denen Antisense-RNA oder Ribozyme transkribiert werden. Ferner ist es auch möglich, Oligonukleotide, z.B. Antisenseoligonukleotide, in die Zelle einzubringen. Antisenseoligonukleotide umfassen vorzugsweise 15 Nukleotide oder mehr. Gegebenenfalls können die Oligonukleotide multimerisiert sein. Ribozyme werden vorzugsweise als Teil eines Genkonstrukts, das stabilisierende Genelemente, z.B. tRNA-Genelemente, enthält, in die Zelle eingeführt. Genkonstrukte dieses Typs sind in der EP A 0 387 775 geoffenbart.

Außer Nukleinsäuremolekülen, die Gene, z.B. virale Gene, aufgrund ihrer Komplementarität inhibieren, können auch Gene mit anderer inhibierender Wirkung verwendet werden. Beispiele dafür sind Gene, die für virale Proteine kodieren, die sog. transdominante Mutationen haben (Herskowitz, 1987). Expression der Gene in der Zelle liefert Proteine, die das entsprechende Wildtypprotein dominieren und auf diese Weise die Zelle, die zelluläre Immunität erwirbt, schützen, indem sie die Virusreplikation verhindern. Geeignet sind transdominante Mutationen viraler Proteine, die für die Replikation und Expression erforderlich sind, z.B. Gag-, Tat- und Rev-Mutanten, von denen gezeigt wurde, daß sie HIV-Replikation inhibieren (Trono et al., 1989; Green et al., 1989; Malim et al., 1989).

Ein anderer Mechanismus zur Erlangung intrazellulärer Immunität umfaßt die Expression von RNA-Molekülen, die die Bindungsstelle für ein essentielles virales Protein enthalten, z.B. sog. "TAR Decoys" (Sullenger et al., 1990).

Beispiele für im Rahmen der somatischen Gentherapie anwendbare Gene, die mit Hilfe der vorliegenden Erfindung als Bestandteil von Genkonstrukten in die Zelle eingeschleust werden können, sind Faktor VIII (Hämophilie A), (siehe z.B. Wood et al., 1984), Faktor IX (Hämophilie B), (siehe z.B. Kurachi et al., 1982), Adenosindeaminase (SCID), (siehe z.B. Valerio et al., 1984), α-1 Antitrypsin (Lungenemphysem), (siehe z.B. Ciliberto et al., 1985) oder das "Cystic fibrosis transmembrane conductance regulator gene" (siehe z.B. Riordan et al., 1989). Diese Beispiele stellen keinerlei Beschränkung dar.

Bezüglich der Größe der Nukleinsäuren sind Anwendungen in einem weiten Bereich möglich; mit Hilfe der vorliegenden Erfindung können Nukleinsäure-Moleküle einer Größenordnung von ca. 0.15 kb (im Falle eines ein Ribozym-Gen enthaltenden tRNA-Gens) bis ca. 50 kb und darüber in die Zelle befördert werden; kleinere Nukleinsäuremoleküle können als Oligonukleotide angewendet werden.

Es ist offensichtlich, daß gerade aufgrund der Tatsache, daß die vorliegende Erfindung keinerlei Beschränkungen hinsichtlich der Gensequenz unterliegt und daß mit Hilfe der Erfindung auch sehr große Genkonstrukte transportiert werden können, breiteste Anwendungsmöglichkeiten gegeben sind. Bei der Bestimmung des molaren Verhältnisses Antikörper-Polykation:Nukleinsäure ist zu berücksichtigen, daß Komplexierung der Nukleinsäure(n) stattfindet. Im Zuge von früheren Erfindungen war festgestellt worden, daß ein Optimum für den Import von Nukleinsäure in die Zelle erzielt werden kann, wenn das Verhälnis Konjugat:Nukleinsäure so gewählt wurde, daß die Internalisierungsfaktor-Polykation/Nukleinsäure-Komplexe weitgehend elektroneutral waren. Es wurde festgestellt, daß die Menge der in die Zelle aufgenommenen Nukleinsäure-Menge nicht verringert wird, wenn ein Teil der Transferrin-Polykation-Konjugate durch nicht-kovalent gebundenes Polykation ersetzt wird; in bestimmten Fällen kann sogar eine beträchtliche Steigerung der DNA-Aufnahme erreicht werden (Wagner et al., 1991a). Dabei war beobachtet worden, daß die DNA innnerhalb der Komplexe in zu toroiden Strukturen mit einem Durchmesser von 80 - 100 nm verdichteter Form vorliegt. Die Menge an Polykation wird somit im Hinblick auf die beiden Parameter Elektroneutralität und Erzielung einer kompakten Struktur gewählt, wobei die Menge an Polykation, die sich im Hinblick auf die Erzielung einer weitgehenden Elektroneutralität der Komplexe, wie sie im Rahmen der vorliegenden Erfindung bevorzugt ist, aus der Ladung der Nukleinsäure ergibt, im allgemeinen auch eine Kompaktierung der DNA gewährleistet.

Eine geeignete Methode zur Bestimmung des Verhältnisses der in den erfindungsgemäßen Komplexe enthaltenen Komponenten besteht darin, zunächst das in die Zellen einzubringende Genkonstrukt zu definieren und, wie oben beschrieben, ein für die jeweilige Transfektion geeeignetes Virus zu bestimmen. Daraufhin wird ein Antikörper, der an das Virus bindet, mit einem Polykation konjugiert und mit dem Genkonstrukt komplexiert. Es kann z.B. so vorgegangen werden, daß ausgehend von einer definierten Virusmenge Titrationen durchgeführt werden, indem die Zielzellen mit dieser (konstanten) Virusmenge und abnehmenden DNA-Komplex-Konzentrationen behandelt werden (oder gegebenenfalls umgekehrt). Auf diese Weise wird das optimale Verhältnis DNA-Komplex:Virus ermittelt. In einem zweiten Schritt werden die Zellen mit abnehmenden Konzentrationen der Virus/DNA-Komplex-Mischung (bei konstantem Verhältnis Virus:Komplex) behandelt und die optimale Konzentration bestimmt. Vorzugsweise ist das Virus ein Adenovirus und das molare Verhältnis von Adenovirus:Nukleinsäure-affiner Substanz ca. 1:1 bis 1:100.

Die Länge des Polykations ist nicht kritisch, sofern die Komplexe im Hinblick auf die bevorzugte Ausführungsform im wesentlichen elektroneutral sind. Der bevorzugte Bereich der Polylysin-Kettenlänge liegt im Bereich von ca. 20 bis ca. 1.000 Lysinmonomere. Es besteht jedoch für eine vorgegebene DNA-Länge keine kritische Länge für das Polykation. Wenn die DNA aus 6.000 bp und 12.000 negativen Ladungen besteht, kann die Menge an Polykation pro Mol DNA z.B. sein:
60 Mol Polylysin 200 oder
30 Mol Polylysin 400 oder
120 Mol Polylysin 100, etc.
Der Durchschnittsfachmann kann mittels einfacher Routineversuche andere Kombinationen von Polykationlänge und molarer Menge auswählen.

Die erfindinngsgemäßen Komplexe können hergestellt werden, indem die Komponenten Nukleinsäure und Antikörper-gekoppeltes Polykation, die jeweils in Form verdünnter Lösungen vorliegen, gemischt werden. Die Herstellung der DNA-Komplexe kann bei physiologischen Salzkonzentrationen erfolgen. Eine weitere Möglichkeit besteht im Einsatz hoher Salzkonzentrationen (etwa 2 M NaCl) und anschließende Einstellung auf physiologische Bedingungen durch langsames Verdünnen bzw. Dialyse. Die jeweils am besten geeignete Reihenfolge für die Mischung der Komponenten Nukleinsäure, Antikörper-Polykation-Konjugat und Virus wird im einzelnen in Vorversuchen bestimmt.

Die Erfindung betrifft in einem weiteren Aspekt ein Verfahren zum Einführen von Nukleinsäure in höhere eukaryotische Zellen, wobei man die Zellen mit den erfindungsgemäßen Komplexen derart in Kontakt bringt, daß eine Internalisierung der Komplexe und deren Freisetzung aus den Endosomen erfolgt.

Die vorliegende Erfindung betrifft in einem weiteren Aspekt pharmazeutische Zubereitungen, enthaltend als wirksame Komponente einen Komplex, bestehend aus therapeutisch wirksamer Nukleinsäure, vorzugsweise als Bestandteil eines Genkonstrukts, und einem über einen Polykation-gekoppelten Antikörper. Bevorzugt liegt diese Zubereitung als Lyophilisat oder in einem geeigneten Puffer in tiefgefrorenem Zustand vor, und die Viruspräparation wird kurz vor der Anwendung mit der Komplex-Lösung vermischt. Gegebenenfalls ist in der pharmazeutischen Zubereitung bereits das Virus enthalten, in diesem Fall liegt sie in tiefgefrorenem Zustand vor. Jeder inerte pharmazeutisch annehmbarer Träger kann verwendet werden, z.B. Kochsalzlösung oder phosphatgepufferte Kochsalzlösung oder jeder Träger, in dem die DNA-Komplexe geeignete Löslichkeitseigenschaften haben, um im Rahmen der vorliegenden Erfindung angewendet zu werden. Bezüglich Methoden zur Formulierung pharmazeutischer Zubereitungen wird auf Remington's Pharmaceutical Sciences, 1980, Bezug genommen.

Gegebenenfalls liegen die für die Transfektion erforderlichen Komponenten, nämlich DNA, Viruspräparation und Antikörper-Konjugat bzw. die Konjugationspartner, gegebenenfalls Internalisierungsfaktor-Konjugat, gegebenenfalls freies Polykation, in einem geeigneten Puffer getrennt oder teilweise getrennt als Bestandteile eines Transfektionskits vor, der ebenfalls Gegenstand der vorliegenden Erfindung ist. Der Transfektionskit der vorliegenden Erfindung umfaßt einen Träger, der einen oder mehrere Behälter, wie Röhrchen, Fläschchen oder ähnliches enthält, die die für die Transfektion der höheren eukaryotischen Zelle entsprechend der vorliegenden Erfindung erforderlichen Materialien enthalten. In einem solchen Transfektionskit kann ein erster Behälter ein oder mehrere verschiedene DNAs enthalten. Ein zweiter Behälter kann ein oder mehrere verschiedene Internalisierungsfaktor-Konjugate enthalten, womit die Anwendung des Transfektionskits als Baukastensystem ermöglicht wird. Ob die Bestandteile als gebrauchsfertige Zubereitung oder getrennt vorliegen, um unmittelbar vor der Verwendung gemischt zu werden, hängt neben der spezifischen Anwendung von der Stabilität der Komplexe ab, die routinemäßig in Stabilitätstests untersucht werden kann.

Die therapeutische Anwendung kann systemisch erfolgen, dabei wird die intravenöse Route bevorzugt. Zielorgane für diese Anwendungsform sind z.B. Leber, Milz, Lunge, Knochenmark, Tumore.

Kürzlich wurde die Möglichkeit aufgezeigt, Myoblasten (unreife Muskelzellen) zu verwenden, um Gene in die Muskelfasern von Mäusen einzubringen. Da von Myoblasten gezeigt wurde, daß sie das Genprodukt ins Blut sezernieren, kann diese Methode breitere Anwendung finden als zur Behandlung genetischer Defekte von Muskelzellen, wie z.B. der Defekt im Zusammenhang mit Muskeldystrophie. Es können somit die manipulierten Myoblasten verwendet werden, um Genprodukte zu liefern, die entweder im Blut wirken, oder vom Blut transportiert werden.

Beispiele für die lokale Anwendung sind das Lungengewebe (Anwendung der erfindungsgemäßen Zusammensetzung als Flüssigkeit zur Instillation oder als Aerosol zur Inhalation), direkte Injektion in die Leber, ins Muskelgewebe oder in einen Tumor oder die lokale Anwendung im Gastrointestinaltakt. Eine weitere Methode für die Verabreichung der pharmazeutischen Zusammensetzung ist die Applikation über das Gallengangsystem. Diese Anwendungsmethode erlaubt den direkten Zugang zu den Hepatozytenmembranen an den Gallenkanälchen, wobei die Wechselwirkung mit Blutbestandteilen vermieden wird.

Die therapeutische Anwendung kann auch ex vivo erfolgen, wobei die behandelten Zellen, z.B. Knochenmarkszellen, Hepatozyten oder Myoblasten in den Körper rückgeführt werden, z.B. Ponder et al., 1991, Dhawan et al., 1991. Eine weitere ex vivo Anwendung der vorliegenden Erfindung betrifft sog. Krebsvaccine. Das Prinzip dieser therapeutischen Möglichkeit besteht darin, Tumorzellen von einem Patienten zu isolieren und die Zellen mit einer für ein Zytokin kodierenden DNA zu transfizieren. In einem nächsten Schritt werden die Zellen gegebenenfalls inaktiviert, z.B. durch Strahlen, derart, daß sie sich nicht länger vermehren, aber noch immer das Zytokin exprimieren. Dann werden die genetisch veränderten Zellen dem Patienten, dem sie entnommen wurden, als Vaccin verabreicht. In der Umgebung der Impfstelle aktivieren die sezernierten Zytokine das Immunsystem, u.a. indem sie zytotoxische T-Zellen aktivieren. Diese aktivierten Zellen können ihre Wirkung in anderen Teilen des Körpers ausüben und greifen auch nichtbehandelte Tumorzellen an. Auf diese Weise wird das Risiko von Tumorrezidiven und Metastasenentwicklung verringert. Eine für die Anwendung von Krebsvaccinen für die Gentherapie geeignete Vorschrift wurde von Rosenberg et al., 1992, beschrieben. Statt der von Rosenberg vorgeschlagenen retroviralen Vektoren kann das Gentransfersystem der vorliegenden Erfindung verwendet werden.

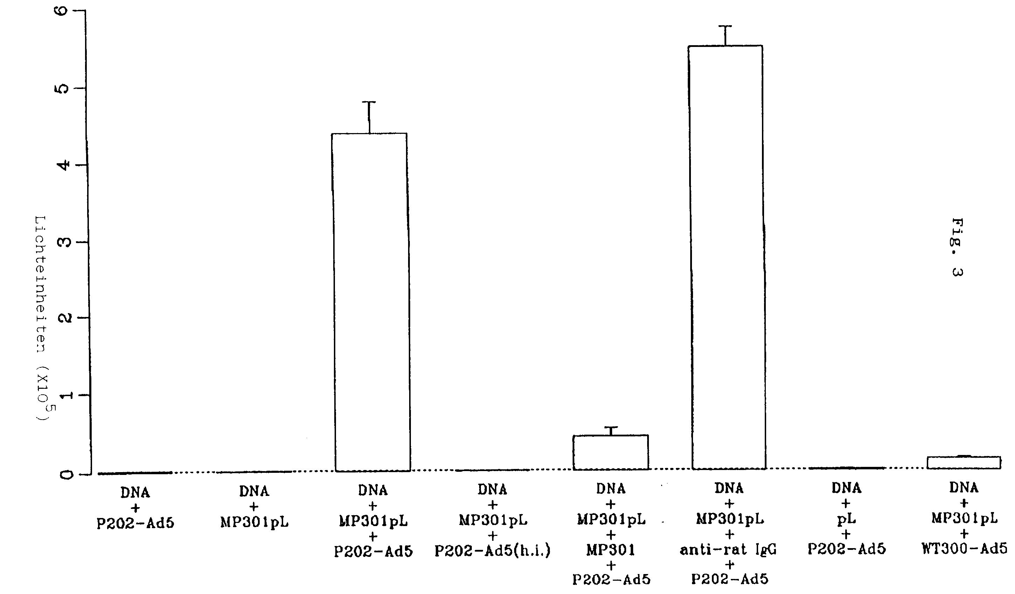

Um die Leistungsfähigkeit von Adenovirus-Antikörper-Polykation/DNA-Komplexe für den Gentransfer festzustellen, wurde als DNA ein Plasmid, enthaltend das für Photinus Pyralis Luciferase kodierende Gen (De Wet et al., 1987) als Reportergen, eingesetzt. Als Zielzellen für die Komplexe wurden HeLa-Zellen verwendet; diese Zellen besitzen eine definierte Population von Zelloberflächenrezeptoren für Adenoviren (Philipson et al., 1968). Wenn die Komponenten des erfindungsgemäßen Konjugats (Virus, Antikörper-Polylysin-Konjugat, DNA) in Kombination angewendet wurden, wurden hohe Werte für die Expression des Luciferase Reportergens erhalten (Fig. 3):
Vergleichsexperimente zeigten, daß das Adenovirus den Transfer von nicht-komplexierter Plasmid-DNA nur geringfügig erhöhte. Außerdem wurde festgestellt, daß DNA, die mit dem Antikörper-gekoppelten Polylysin (ohne Bindung an das Virus) komplexiert war, nicht nennenswert in HeLa-Zellen aufgenommen wurde. In starkem Gegensatz dazu konnten mit dem Komplex hohe Gen-Expressionswerte erreicht werden, wenn die DNA durch Bindung über den Antikörper mit dem Adenovirus in Wechselwirkung treten konnte. Dieser Effekt wurde aufgehoben, wenn die Virionen vor der Komplexbildung hitzebehandelt wurden. Da diese Behandlung selektiv die viralen Aufnahmefunktionen aufhebt, ohne die strukturelle Integrität des Virus zu stören (Defer et al., 1990), kann aus diesen Experimenten geschlossen werden, daß es die spezifischen Aufnahmefunktionen des Adenovirus sind, die den entscheidenden Beitrag für die Leistungsfähigkeit des Gentransfers darstellen. Weiters wurde festgestellt, daß die Kompetition um das heterologe Epitop auf der Oberfläche des chimären Adenovirus durch einen spezifischen, nicht Polylysingebundenen monoklonalen Antikörper ebenso eine Verringerung der Netto-Genexpression bewirkt. Dieser Effekt trat nicht auf, wenn ein unspezifischer Antikörper eingesetzt wurde. Es ist daher die spezifische Wechselwirkung zwischen der Antikörpergebundenen DNA und dem korrespondierenden Adenovirus-Oberflächenepitop wesentlich für die Gewährleistung eines funktionellen Gentransports durch den Komplex. Weiters wurde festgestellt, daß Polylysin-komplexierte DNA nicht nennenswert durch das Adenovirus in die Zielzellen befördert wurde. Dies ist ein Hinweis dafür, daß die Gentransfer-Kapazität der Komplexe nicht auf der Kondensierung von DNA beruht, sondern von der Antikörper-vermittelten Bindung des Reportergens an das Virion abhängt.

In Übereinstimmung damit konnte die Verwendung eines Virus, der das vom Polylysin-gekoppelten Antikörper erkannte Epitop nicht aufwies, die hohen Gen-Expressionswerte, die durch ein Virus erreicht wurden, das dieses Epitop besaß, nicht erzielen. Dieses Virus konnte jedoch das Ausmaß des Gentransfers über Backgroundniveau erhöhen. Da von Adenoviren bekannt ist, daß sie die zelluläre Aufnahme von Makromolekülen über die Flüssigphase unspezifisch verstärken können (Defer et al., 1990), war dieses Ergebnis nicht unerwartet. Die Tatsache, daß dieser unspezifische Transport eine deutlich geringere Expression des Reportergens bewirkte als das spezifische Virus, das an den Antikörper-Polylysin/DNA-Komplex binden konnte, zeigt die Wichtigkeit der spezifischen Bindung der Komplexkomponenten.

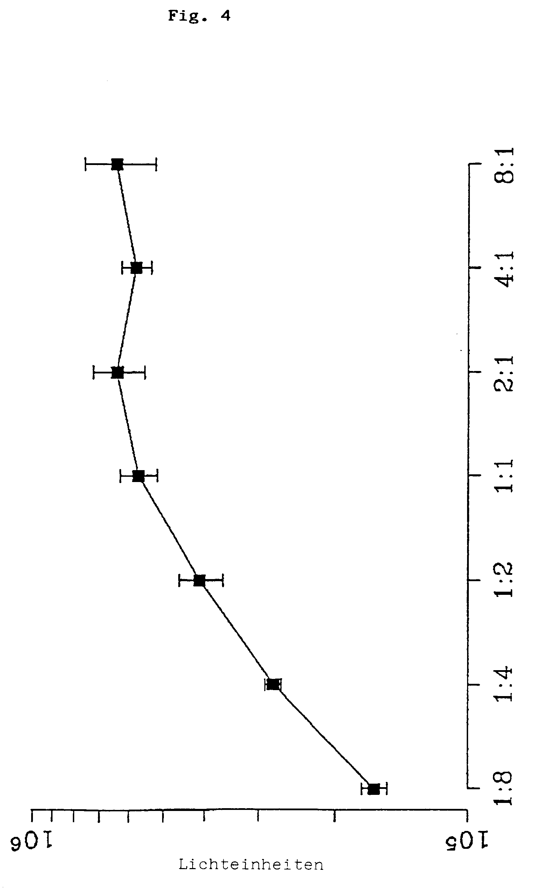

Die Wechselwirkung von Plasmid-DNA mit Polylysin-Konjugaten resultiert in signifikanten strukturellen Änderungen des DNA-Moleküls, die am deutlichsten durch eine auffällige Kondensation zu einer toroiden Struktur von 80 - 100 nm charakterisiert wird (Wagner et al., 1991a). Der Durchmesser des Virus liegt in einer Größenordnung von 70 - 80 nm (Philipson, 1983). Es wurde daher auf der Grundlage von sterischen Überlegungen davon ausgegangen, daß das optimale Verhältnis von Adenovirus zu Antikörper-Polylysinkomplexierter DNA nicht größer als 1 : 1 sein sollte. Darüberhinaus ist der Durchmesser der "coated pits", durch die der anfängliche Aufnahmeschritt der Rezeptorvermittelten Endozytose herbeigeführt wird, ca. 100 nm (Darnell et al., 1975). Aufgrund dieser Tatsache wurde angenommen, daß Multimere, die diese Größe überschreiten, in ihrer Aufnahmefähigkeit eingeschränkt sein würden. Im Rahmen der vorliegenden Erfindung wurden diese Zusammenhänge analysiert, wobei durch Anwendung von Adenovirus in molarem Überschuß gegenüber der Antikörper-Polylysin-komplexierten DNA gezeigt wurde, daß ein Maximum an Reportergen-Expression bei einem Verhältnis von 1 : 1 bewirkte (Fig. 4). Es wurde daher im Rahmen der durchgeführten Versuche als optimales Konjugat ein solches ermittelt, das aus einer einzigen Adenovirus-Internalisierungdomäne in Verbindung mit einer einzigen Antikörper-Polylysin/DNA-Bindungsdomäne besteht.

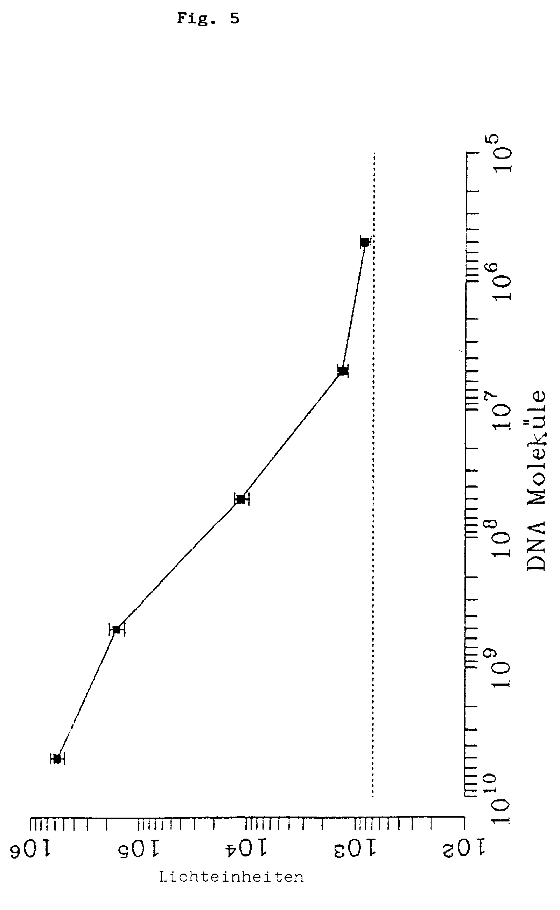

Als nächstes wurde die Leistungsfähigkeit des Gentransfers von Adenovirus-Antikörper-Polykation-Konjugaten, die dieses optimierte Verhältnis aufweisen, untersucht. Wenn logarithmische Verdünnungen des Komplexes den Zielzellen beigegeben wurden, ergab sich eine entsprechende logarithmische Abnahme der Reportergen-Expression (Fig. 5), wobei auffallend war, daß 10⁷ DNA-Moleküle, die mittels dieses Vektorsystems auf 10⁶ HeLa-Zellen angewendet wurden, eine nachweisbare Expression des Reportergens bewirkten. Überraschenderweise konnte somit mit so wenigen wie 10 DNA-Molekülen pro Zelle in Form von Adenovirus-Polykation-DNA-Komplexen eine effiziente Expression eines Fremdgens herbeigeführt werden.

Es zeigt sich somit hinsichtlich der Größenordnung der Aufnahme von DNA eine deutliche Überlegenheit der erfindungsgemäßen Konjugate gegenüber den DNA-Gentransfer-Vektoren, von denen größenordnungsmäßig 500.000 DNA-Moleküle pro Zelle benötigt werden (Felgner et al., 1987, Felgner et al., 1989, Maurer, 1989). Da diese Methoden den größten Teil der DNA effizient in den Zielbereich der Zelle, nämlich das Zytosol, befördern (Felgner et al., 1989, Malone et al., 1989, Loyter et al., 1982), beruht die Leistungsfähigkeit der erfindungsgemäßen Konjugate möglicherweise nicht ausschließlich auf der Verstärkung der Freisetzung der Fremd-DNA ins Zytoplasma; es dürften zusätzlich noch weitere Vorgänge auf dem Weg des Gentransfers verstärkt werden.

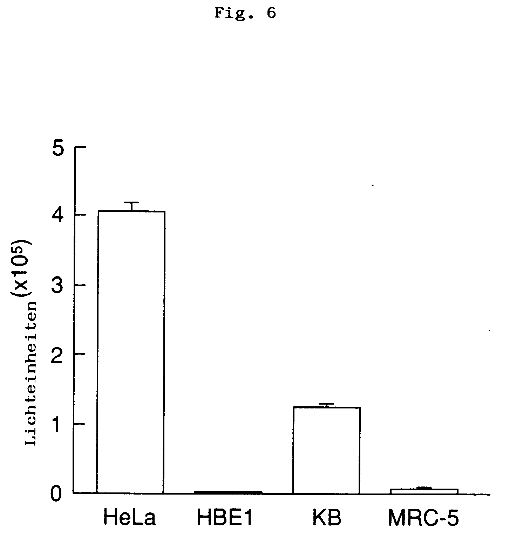

In der Konfiguration der Adenovirus-Polylysin-DNA-Komplexe fungiert der Adenovirusanteil sowohl als Mittel für das Aufbrechen von Endosomen als auch als Liganden-Domäne des Komplexes. Es sollte daher die Gentransfereffizienz der Komplexe für eine gegebene Zielzelle die relative Zahl von Adenovirus-Zelloberflächenrezeptoren wiederspiegeln. Sowohl die Zellinie HeLa als auch die Zellinie KB, die große Mengen an Adenovirusrezeptoren besitzen (Philipson et al., 1968), zeigten einen ensprechend hohen Grad an Zugänglichkeit für den Gentransfer mittels Adenovirus-Polylysin-DNA-Komplexen (siehe Fig. 6). Im Gegensatz dazu wird die relativ niedrige Anzahl von Adenovirusrezeptoren, die die Zellinien MRC-5 (Precious und Russell, 1985) und HBE1 charakterisiert, in einem niedrigen Gentransferniveau mittels den Komplexen in diese Zellen reflektiert.

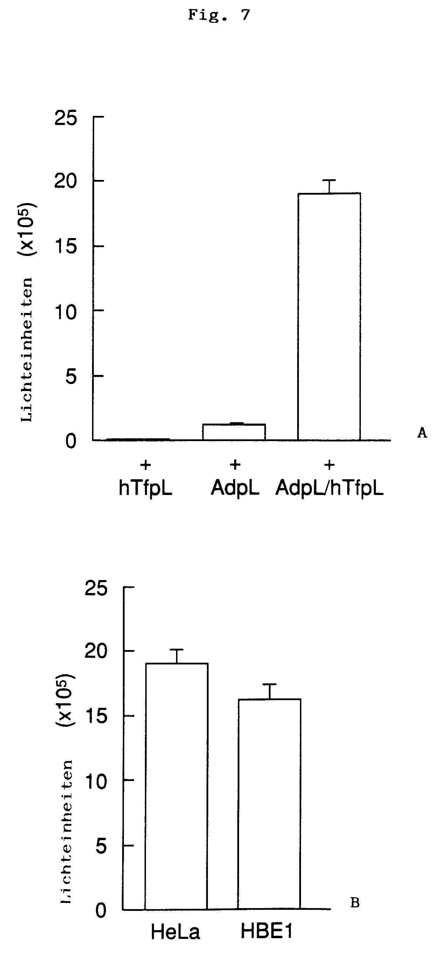

Ternäre Komplexe, die zusätzlich zum Adenovirus einen anderen Zelloberflächenliganden (Internalisierungsfaktor) enthielten, zeigten signifikant höhere Werte als die Konjugate, die nur Transferrin- oder Adenovirus-Ligandendomänen hatten (Fig. 7A). Die Größenordnung dieser Verstärkung beruhte deutlich nicht auf der Grundlage eines additiven Effekts von Transferrin-Polylysin-DNA-Komplexen plus Adenovirus-Polylysin-DNA-Komplexen. Da die ternären Komplexe über den Adenovirus- oder Transferrin-Aufnahmemechanismus internalisiert werden können, läßt dieses offensichtliche Zusammenwirken vermuten, daß die Adenovirusdomäne den Eintritt in die Zelle über beide Wege erleichtert, vermutlich auf der Grundlage der durch Adenovirus bewirkten Endosomolyse. Um die selektive Mitwirkung der adenoviralen Domäne des ternären Komplexes beim Aufbrechen von Endosomen zu zeigen, wurden die Kombinations-Komplexe auf Zellinien aufgebracht, die unterschiedliche Empfänglichkeit für Adenovirus-Polylysin-DNA-Komplexe aufweisen (Fig. 7B). Die epitheliale Atemwegszellinie zeigt im Vergleich zu HeLa-Zellen sehr niedrige Gentransferwerte, erzielt durch AdpL-DNA-Komplexe (Fig. 6), was die relativ niedrige Zelloberflächenpopulation an Adenovirusrezeptoren, die für diese Zellinie charakteristisch ist, wiederspiegelt. In deutlichem Gegensatz führte die Verwendung von ternären AdpL/TfpL-Komplexen zu Genexpressionsniveaus, die vergleichbar mit den in HeLa-Zellen gesehenen waren. Die Empfänglichkeit dieser Zellinie für den Gentransfer durch die ternären Komplexe stimmt mit dem Konzept überein, daß die Adenovirusdomäne über den Transferrin-Mechanismus aufgenommen wird, wobei sie den Gentransfer verstärkt, indem sie den Aufbruch von Endosomen bewirkt. Daraus ergibt sich die Möglichkeit, die endosomolytische Eigenschaft von Adenovirus und anderen Viren bei der Konstruktion von Konjugaten auszunutzen, die dadurch die Fähigkeit erhalten, dem Zellvesikelsystem zu entkommen.

Im Rahmen der vorliegenden Erfindung wurde ferner in einem Nagetiermodell der direkte in vivo Transfer eines Gens in das Atemwegsepithel mit Hilfe der erfindungsgemäßen Komplexe gezeigt. Dies begründet die Möglichkeit, die vorliegende Erfindung anzuwenden, um eine transiente Genexpression im Atemwegsepithel zu erzielen. Die Möglichkeit, eine genetische Modifikation von Atemwegszellen in situ zu erreichen, stellt eine mögliche Strategie der Gentherapie für Erkrankungen des Atemwegsepithels dar. Bei den durchgeführten Versuchen ergaben die Transferrin-Polylysin-DNA-Komplexe ein geringes Reportergen-Expressionsniveau. Dies ist im Einklang mit der Tatsache, daß diese Art von Konjugat in den Endosomen eingeschlossen werden dürfte. Die binären Adenovirus-Polylysin-Komplexe bewirkten eine signifikant höhere Genexpression. Diese wurde weiter erhöht, indem ein zweiter Internalisierungsfaktor in dem Kombinations-Komplex hTfpL/AdpL eingesetzt wurde. Um festzustellen, ob die Nettogenexpression mit der Transduktionshäufigkeit übereinstimmt, wurde der Anteil an Zellen, die mit den diversen Komplexarten transduziert wurden, ermittelt. Es wurde festgestellt, daß eine solche Übereinstimmung vorliegt; das mit hTfpL modifizierte Atemwegsepithel in der Primärkultur zeigte < als 1 % Transduktionshäufigkeit, die AdpL-Komplexe im Bereich von 20 - 30 % und die Kombinations-Komplexe mehr als 50 % modifizierte Zellen.

Die in vivo im Nagetiermodell durchgeführten Experimente stimmten mit den in der Primärkultur erhaltenen Ergebnissen überein. Die Untersuchung von histologischen Lungenabschnitten von Ratten, die mit lacZ-Kombinations-Komplexen behandelt worden waren, zeigten ungleichmäßige Zonen von β-Galaktosidaseaktivität, die zahlreiche positive Zellen enthielten. Die positiven Regionen wurden den bronchiolen und distalen Atemwegsregionen zugeordnet.

### Figurenübersicht

- Fig. 1:: Schematische Darstellung von Adenovirus-Polykation-DNA-Komplexen, enthaltend ein Fremdepitop auf dem Adenovirus-Kapsid
- Fig. 2:: Herstellung des chimären Adenovirus Ad5-P202
- Fig. 3:: Gentransfer mittels Adenovirus-Polykation-DNA-Komplexen in HeLa-Zellen
- Fig. 4:: Bestimmung des optimalen Verhältnisses von Adenovirus und Polylysin-Antikörperkomplexierter DNA
- Fig. 5:: Bestimmung der Leistungsfähigkeit des Gentransfers durch Adenovirus-Polykation-DNA-Komplexe
- Fig. 6:: Gentransfer mittels Adenovirus-Polykation-DNA-Komplexen in verschiedene eukaryotische Zellen
- Fig. 7:: Gentransfer mittels Kombinations-Komplexen, enthaltend Adenovirus- und Humantransferrin-Konjugate A: Vergleich der Effizienz von Kombinations-Komplexen mit binären Komplexen in HeLa-Zellen B: Vergleich von HeLa-Zellen und HBE1-Zellen hinsichtlich der Gentransfereffizienz von Kombinations-Komplexen
- Fig. 8:: Dreidimensionale Darstellung der Hexon-Untereinheit; mögliche Insertionsstellen in der Ad5-Hexongensequenz, erhalten mittels gerichteter Mutagenese
- Fig. 9:: Transfektion von primären Atemwegs-Epithelzellkulturen. Relative Höhe des Nettogentransfers
- Fig. 10:: Transfektion von primären Atemwegs-Epithelzellkulturen. Relative Transduktionshäufigkeit
- Fig. 11:: Gentransfer über die intratracheale Route in vivo. Relative Höhe des Nettogentransfers in vivo
- Fig. 12:: Gentransfer über die intratracheale Route in vivo. Lokalisierung der heterologen Genexpression im Atemwegsepithel
- Fig. 13:: In vivo Anwendung von chimären Adenovirus-Lectin-Polylysin-DNA-Komplexen

Die Erfindung wird anhand der folgenden Beispiele illustriert:

### Beispiel 1

### Herstellung von Antikörper-Polylysin-Konjugaten

### 1) Herstellung des chimären Adenovirus Ad-P202

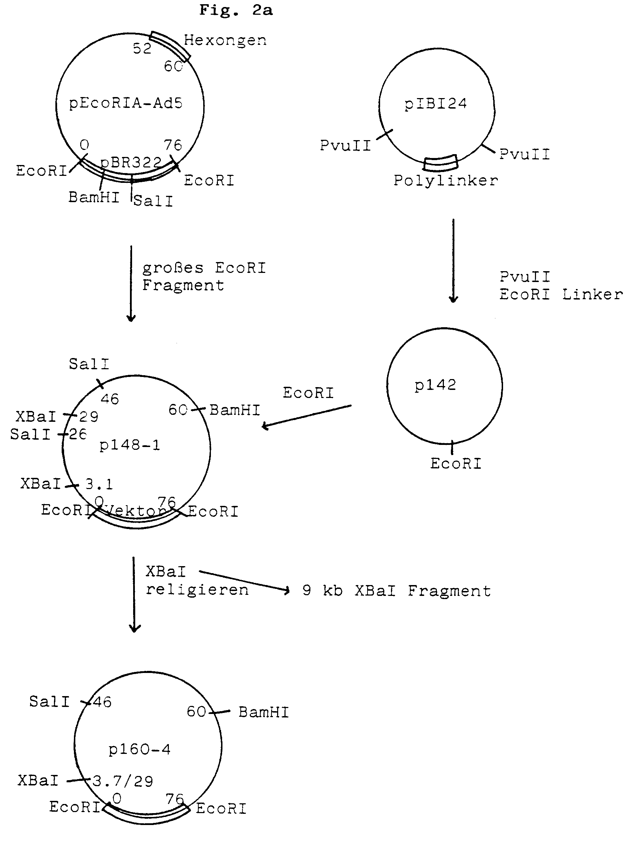

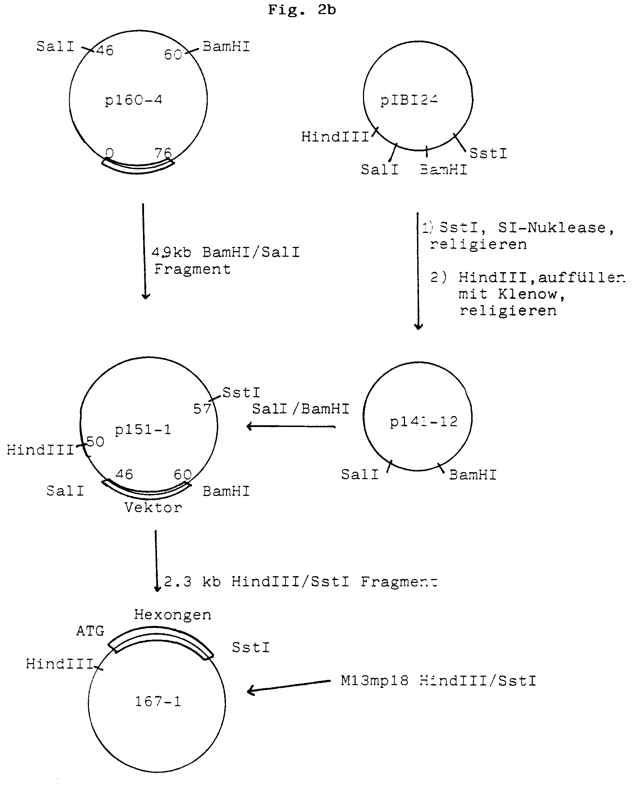

Um Änderungen im Ad5 Hexon-Gen vorzunehmen, war es zunächst notwendig, das Gen zu subklonieren. Das Plasmid pEcoRIA-Ad5 (Berkner und Sharp, 1983) enthält den linken Teil des Adenovirus-Genoms von map unit (m.u.) 0 - 76. Das Hexon-Gen liegt zwischen m.u.52 und m.u.60. Ein 2.3 kbp HindIII/SstI-Fragment enthält den Teil des Hexon-Gens, an dem die Änderung vorgenommen werden sollte. Da in pEcoRIA-Ad5 mehrere HindIII und SstI-Stellen enthalten sind, war es erforderlich, einige Zwischen-Plasmide zu konstruieren, um das geänderte Hexon-Gen wieder im Originalplasmid zusammensetzen zu können. Ein SalI/BamHI Fragment (m.u. 46 - 60) enthält das Hexon-Gen ohne zusätzliche HindIII- oder SstI-Stellen. Zuerst wurde die Adenovirus-DNA von m.u. 0 - 76 rekloniert, indem ein Vektor der Bezeichnung p142 (abgeleitet von dem käuflich erhältlichen Plasmid pIBI24 (IBI, Inc.) durch Restriktionsverdau mit PvuII, gefolgt von der Insertion eines EcoRI-Linkers), verwendet wurde, der keine SstI- oder BamHI-Stellen aufweist. Dann wurden die SalI-Stellen bei m.u. 26 entfernt, indem das XbaI-Fragment (m.u. 3.7 - 29) entfernt wurde; der erhaltene Vektor wurde mit p141-12 bezeichnet. Abschließend wurde das gewünschte HindIII/SstI-Fragment in M13mp18 kloniert und war somit bereit für die Mutagenese.
Gerichtete Mutagenese (site directed mutagenesis) wurde mit einem der erhaltenen Klone nach der von Kunkel, 1985, beschriebenen Methode durchgeführt. Dabei wurden die Codons 188 - 194 des Hexon-Gens entfernt und an dieser Stelle eine nur einmal vorkommende PmlI-Stelle eingeführt. Der erhaltene Klon (167-1) wurde daraufhin mit PmlI geschnitten und ein doppelsträngiges Oligonukleotid, kodierend für die Aminosäuren 914 - 928 des Mycoplasma pneunmoniae P1-Proteins, eingesetzt (Inamine et al., 1988). Diese P1-Sequenz enthält ein Epitop, das vom monoklonalen Antikörper 301, dessen Herstellung im folgenden beschrieben wird, erkannt wird. Das veränderte HindIII/SstI-Fragment wurde aus p167-1 isoliert und wieder in das Ursprungsplasmid pEcoRIA-Ad5 hineinligiert. Die Herstellung von Ad-P202 ist in Fig. 2 dargestellt.

### 2) Herstellung eines monoklonalen Antikörpers mit Spezifizät für das chimäre Adenovirus (MP301)

### a) Immunisierung

Die Herstellung des monoklonalen Antikörpers erfolgte nach Standardmethoden.
Als Antigen wurde der Mycoplasma pneumoniae Stamm M-129 (ATCC#29342) verwendet. Nach Züchtung in einer Kulturflasche (Hu et al., 1977) wurde 3 mal mit PBS gewaschen, Mycoplasma pneumoniae geerntet und in 0.5 ml PBS aufgenommen. 10 µg des Antigens wurden für die Immunisierung verwendet:
3 ca. 6 Wochen alte weibliche BALB/c Mäuse wurden nach folgendem Schema immunisiert:
- 1.Imminnisierung:: ca. 10 µg Antigen pro Maus in komplettem Freund'schem Adjuvans intraperitoneal.
- 2.Immunisierinng:: ca. 10 µg Antigen pro Maus in inkomplettem Freund'schem Adjuvans subkutan, 3 Wochen nach der 1. Immunisierung.
- 3.Immunisierung:: ca. 10 µg Antigen pro Maus in inkomplettem Freund'schem Adjuvans intraperitoneal, 2 Wochen nach der

### 2. Immunisierung.

Eine Woche danach wurden den Mäusen Serumproben entnommen und die Serumtiter bestimmt. Die Maus mit dem höchsten Titer wurde durch iv-Injektion in den Schwanz mit 10 µg Antigen geboostert; die Milzzellen dieser Maus wurden nach 3 Tagen für die Fusion mit Hybridomzellen präpariert.

### b) Fusion:

Ca. 10⁸ Milzzellen wurden nach der Methode von Köhler und Milstein, 1975, in Gegenwart von PEG 4000 (50% in serumfreiem Kulturmedium) mit ca. 10⁸ Myelomzellen der Linie SP2/0 Ag14 (ATCC CRL-1581) fusioniert. Danach wurden die Zellen 2 Wochen in HAT-Selektionsmedium, daraufhin eine Woche in HT-Medium, abschließend in normalem Kulturmedium (DMEM plus 10% FCS plus Penicillin, Streptomycin) gezüchtet. Mit Hilfe des Radioimmunosorbent Assay (RIA) wurde nach Antikörper produzierenden Klonen gescreent, die Spezifität für das Mycoplasma pneumoniae P1 Protein wurde mittels Western Blot bestimmt. Um Monoklone zu erhalten, wurde die "Soft Agar" Methode angewendet.

### c) Untersuchung des monoklonalen Antikörpers MP301 auf neutralisierende Wirkung von Adenovirus Ad-P202

Um festzustellen, ob der monoklonale Antikörper MP301 die Fähigkeit des Virus, Zellen zu infizieren, neutralisiert, wurde der Titer von Ad-P202 einmal mit und einmal ohne Antikörperzugabe (7µg/ml) bestimmt, wobei als Zielzellen HeLa-Zellen (ca. 50% konfluent in 2% FCS/DMEM auf 96-Loch-Platten) verwendet wurden. Von Ad-P202 wurden Serienverdünnungen hergestellt und auf die HeLa-Titerplatten mit oder ohne Antikörper aufgebracht. Die Platten wurden 48 h bei 37°C, 5% CO₂ inkubiert, mit Kristallviolett gefärbt und auf IC 50 ("inhibition concentration", ca. 50% Zell-Lysis) untersucht. Es wurde mit und ohne Antikörper der Titer von 1:2048 erhalten.

### d) Herstellung von MP301-Polylysin-Konjugaten

Die Kopplung des monoklonalen Antikörpers mit Polylysin wurde nach der von Wagner et al., 1990, und in der EP-A1 388 758 beschriebenen Methode durchgeführt.

20.6 nmol (3.3 mg) des monoklonalen Antikörpers MP301 in 1 ml 200mM HEPES pH 7.9 wurden mit einer 5 mM ethanolischen Lösung von SPDP (100 nmol) behandelt. Nach 3 h bei Raumtemperatur wurde der modifizierte Antikörper über eine Sephadex G-25 Säule gelfiltriert, wobei 19 nmol Antikörper, modifiziert mit 62 nmol Dithiopyridinlinker, erhalten wurde. Der modifizierte Antikörper wurde mit 3-Mercaptopropionat-modifiziertem Polylysin (22 nmol, durchschnittliche Kettenlänge 300 Lysinmonomere, FITC-markiert, modifiziert mit 56 nmol Mercaptopropionat-Linker) in 100 mM HEPES pH 7.9 unter Argonatmosphäre reagieren gelassen. Konjungate wurden mittels Kationenaustauschchromatographie auf einer Mono S HR5-Säule (Pharmacia) isoliert. (Gradient: 20 - 100 % Puffer. Puffer A: 50 mM HEPES pH 7.9; Puffer B: Puffer A plus 3 M Natriumchlorid. Die Produktfraktion eluierte bei einer Salzkonzentration zwischen 1.65 M und 2 M. Dialyse gegen HBS (20mM HEPES pH 7.3, 150 mM NaCl) ergab ein Konjugat, bestehend aus 9.1 nmol MP301 und 9.8 nmol Polylysin.

### Beispiel 2

### Gentransfer mittels Adenovirus-Polykation-DNA-Komplexen in eukaryotische Zellen

Im Zuge der in diesem Beispiel durchgeführten Experimente wurden verschiedene Kombinationen spezifischer und nichtspezifischer Komplexkomponenten auf ihre Fähigkeit, ein Reportergen in HeLa- und in andere Zellen transportieren zu können, untersucht.

Die Komplexierung von DNA mit dem Antikörpergekoppelten Polylysin wurde vorgenommen, indem 6 µg gereinigter pRSVL-DNA in HBS (20 mM HEPES, 150 mM NaCl, pH 7.3) auf ein Gesamtvolumen von 350 µl verdünnt und mit 9.5 µg MP301pL in 150 µl Gesamtvolumen desselben Puffers vereinigt wurden. (pRSVL enthält das Photinus pyralis Luciferasegen unter der Kontrolle des Rous Sarcoma Virus LTR Enhancer/Promoters (Uchida et al., 1977, De Wet et al., 1987), hergestellt mittels Triton-X Lyse Standard-Methode (Maniatis), gefolgt von CsCl/EtBr Gleichgewichtsdichtegradienten-Zentrifugation, Entfärbung mit Bintanol-1 und Dialyse gegen 10 mM Tris/HCl pH 7,5, 1 mM EDTA in 350 µl HBS (150 mM NaCl, 20 mM HEPES pH 7.3).) Die Menge von Antikörper-gekoppeltem Polylysin basiert auf Berechnung der Menge, die nötig ist, um Elektroneutralität der importierten DNA zu erreichen. Die Polylysin-Antikörper-komplexierte DNA wurde in HBS auf eine Endkonzentration von 2 x 10¹¹ DNA-Moleküle/ml verdünnt. Das Adenovirins P202-Ad5 wurde in eiskaltem DMEM, ergänzt mit 2 % FCS, auf eine Endkonzentration von 2 x 10¹¹ Viruspartikel/ml verdünnt. Gleiche Volumina von Antikörper-Polylysin-DNA und Virus wurden vereinigt und 30 min bei Raumtemperatur inkubiert. Als Zielzellen für den Gentransfer wurden HeLa-Zellen verwendet, die in DMEM-Medium, ergänzt mit 5 % FCS, 100 I.E. Penicillin/ml und 100 µg Streptomycin/ml, in 60 mm Gewebekulturschalen gezüchtet wurden (300.000 Zellen). Zum Vergleich mit HeLa-Zellen wurden die Zellinien HBE1, KB (ATCC Nr. CCL 17) und MRC-5 (ATCC Nr. CCL 171) untersucht. HBE1, eine Atemwegszellinie, wurde in F12-7X Medium, wie von Willumsen et al., 1989, beschrieben, gezüchtet. KB und MRC-5 wurden in EMEM-Medium/10 % hitzeinaktiviertes FCS/100 I.E. Penicillin pro ml/100 µg Streptomycin pro ml/10 mM nicht-essentielle Aminosäuren/2 mM Glutamin gezüchtet.

Vor Anwendung des Transfektionsmediums wurden die Platten bei 4°C 30 min lang gekühlt, das Medium entfernt, 1 ml Transfektionsmedium beigegeben und die Zellen 2 h lang bei 4°C inkubiert. Dieser Schritt wurde durchgeführt, um die Bindung der DNA-Komplexe an die Zellen zu bewirken, ohne daß sie schon internalisiert werden. Nach diesem Bindungsschritt wurden die Platten 3 x mit eiskaltem 2 % FCS/DMEM gewaschen, um nichtgebundene Reaktionskomponenten in der Flüssigphase zu entfernen. Nach Zusatz von 2 ml eiskaltem 2 % FCS/DMEM wurden die Platten langsam erwärmen gelassen. Daraufhin wurden die Platten für 16 h in den Brutschrank gegeben (37°C, 5 % CO₂). Um die Reportergen-Expression zu messen, wurden Zellysate hergestellt, auf Gesamtproteingehalt standardisiert und, genau wie von Zenke et al., 1990, beschrieben, auf Luciferase-Aktivität untersucht. (Das Luminometer war so geeicht worden, daß ein Picogramm Luciferase 50.000 Lichteinheiten liefert.)

pRSVL-Reporterplasmid-DNA wurde mit Adenovirus P202-Ad5 vereinigt, ohne vorher mit dem Polylysin-Antikörper-Konjugat komplexiert worden zu sein (DNA + P202-Ad5). Weiters wurde pRSVL-DNA, komplexiert mit dem Antikörper-gekoppelten Polylysin, in Abwesenheit des spezifischen Virus (DNA + MP301pL) untersucht und diese beiden Reaktionsmedien mit einem Reaktionsmedium, das die vollständige Kombination der Komplexkomponenten enthielt (DNA + MP301pL + P202Ad5), verglichen. In analoger Weise wurden die Komplexe auf ihre Leistungsfähigkeit für den Gentransfer untersucht, indem ein spezifischer Antikörper verwendet wurde, der vor der Komplexbildung hitzeinaktiviert (50°C, 30 min) worden war (DNA + MP301pL + P202-Ad5). Es wurden Kompetitionsexperimente durchgeführt, indem das spezifische Adenovirus in Gegenwart des Polylysingekoppelten Antikörpers MP301 plus ein zehnfacher molarer Überschuß von nicht-Polylysin-gekoppeltem MP301 (DNA + MP301pL + MP301 + P202-Ad5) oder in Gegenwart von MP301pL und einem zehnfachen molaren Überschuß von nicht-gekoppeltem irrelevanten monoklonalen Antikörper, Anti-Ratten-IgG (DNA + MP301pL + anti-rat IgG + P202-AD5). Weiters wurde vor der Inkubation mit dem spezifischen Virus die Reporterplasmid-DNA mit nichtkonjugiertem Polylysin (4 µg) in einer Menge, äquimolar dem Antikörper-gekoppelten Polylysin, komplexiert (DNA + pL + P202-Ad5). Die Komplexbildungsreaktionen unter Verwendung des Adenovirus WT300, dem das von MP301 erkannte Epitop fehlt, wurden genauso durchgeführt, wie für das spezifische Virus P202-AD5. Die Experimente wurden sämtlich dreifach durchgeführt. Die Ergebnisse mit HeLa-Zellen sind in Fig. 3 dargestellt; die Daten stellen die Durchschnittswerte ± SEM dar. Die strichlierte horizontale Linie zeigt die Background-Luciferaseaktivität von nichtbehandelten HeLa-Zellen. Die mit den Zellinien HBE1, KB und MRC-5 im Vergleich zu HeLa-Zellen erzielten Ergebnisse sind in Fig. 6 dargestellt.

### Beispiel 3

### Bestimmung des optimalen Verhältnisses von Adenovirus und Antikörper-Polylysin/DNA für den Gentransfer

In den durchgeführten Versuchen, deren Ergebnis in Fig. 4 dargestellt ist, wurden Adenovirus-Antikörper-Polylysin/DNA-Komplexe bei verschiedenen Verhältnissen der Komplexkomponenten auf ihre Fähigkeit, den Gentransfer in HeLa-Zellen zu ermöglichen, untersucht. Die Komplexbildungsreaktionen wurden durchgeführt, wie in Beispiel 2 angegeben, mit dem Unterschied, daß 2.5 x 10¹⁰ DNA-Moleküle, komplexiert mit dem Antikörper-Polylysin-Konjugat, eingesetzt wurden, wobei unterschiedliche Mengen des spezifischen Adenovirus P202-Ad5 zur Anwendung kamen. Das Züchten der Zellen, die Aufbringung der Komplexe auf die Zellen, die Inkubation der Zellen und die Bestimmung der Reportergen-Expression war analog Beispiel 2. Die gezeigten Daten stellen den Durchschnittswert ± SEM von vier verschiedenen Experimenten dar.

### Beispiel 4

### Bestimmung der Leistungsfähigkeit des Gentransfers von Adenovirus-Polykation-DNA-Komplexen

Limitierende Verdünnungen des Komplexes, der genau wie in Beispiel 2 hergestellt wurde, wurden daraufhin untersucht, wie wirksam sie sind, eine nachweisbare Expression des Reportergens in HeLa-Zellen zu ermöglichen. Nach der Komplexbildung wurden logarithmische Verdünnungen des Komplexes in 2 % FCS/DMEM vorgenommen. 1 ml Aliquots der verschiedenen Verdünnungen wurden auf 60 mm Gewebekulturschalen, die 5 x 10⁵ HeLa-Zellen enthielten, aufgebracht. Nach einstündiger Inkubation (37°C, 5 % CO₂) wurden 3 ml 5 % FCS/DMEM zugefügt und die Platten weitere 16 h lang unter denselben Bedingungen inkubiert. Die Bestimmung der Reportergen-Expression wurde wie in Beispiel 2 durchgeführt. Die in Fig. 5 angegebenen Werte für die Luciferase-Expression entsprechen den Durchschnittswerten ± SEM von 3 bzw. 4 Versuchen. Die strichlierte horizontale Linie zeigt die Background-Luciferaseaktivität von unbehandelten HeLa-Zellen.

### Beispiel 5

### Gentransfer mittels Kombinations-Komplexen, enthaltend Adenovirus- und Humantransferrin-Konjugate

Um die ternären Komplexe, die eine Kombination von Adenovirus- und Humantransferrin-Domänen enthalten, herzustellen, wurde das mit einem Epitop versehene Adenovirus P202-Ad5 (2.5 x 10¹⁰ Partikel) in 750 µl 2 % FCS/DMEM verdünnt und mit dem Polylysin-gekoppelten monoklonalen Antikörper MP301pLys (2 µg) in 250 µl HBS vereinigt. Die Inkubation wurde für 30 min bei Raumtemperatur vorgenommen. Daraufhin wurde Plasmid-DNA pRSVL (6 µg), verdünnt in 250 µl HBS, der Mischung beigegeben und weitere 30 min lang bei Raumtemperatur inkubiert. Von den daraus resultierenden Adenovirus-Polylysin-DNA-Komplexen wurde angenommen, daß sie auf Basis des Gesamtpolylysingehalts unvollständig kondensierte DNA enthalten. Um die DNA-Kondensation zu vervollständigen und den Komplexen eine Humantransferrin-Domäne einzuverleiben, wurden 9 µg Humantransferrin-Polylysin-Konjugate (Wagner et al., 1990), verdünnt in 250 µl HBS, zu den Adenovirus-Polylysin-DNA-Komplexen gegeben. Abschließend wurde 30 min bei Raumtemperatur inkubiert. Mit den erhaltenen Kombinations-Komplexen wurden Gewebekulturzellen inkubiert, wie beschrieben, um spezifische Bindung der gebildeten Komplexe zu erreichen (4°C, 2 h). Die Platten wurden dann 3 x mit eiskaltem 2 % FCS/DMEM gewaschen und nach Zugabe von 2 ml 2 % FCS/DMEM für 16 h in den Brutschrank (37°C, 5 % CO₂) gegeben. Die Messung der Reportergenexpression erfolgte, wie beschrieben. Fig. 7A zeigt die relativen Werte für die Genexpression, bewirkt durch Humantransferrin-Polylysin-DNA-Komplexe (hTfpL), Adenovirus-Polylysin-DNA-Komplexe (AdpL) und ternäre in HeLa-Zellen. Fig. 7B zeigt die relative Zugänglichkeit für den Gentransfer durch ternäre Komplexe (AdpL/hTfpL) von HeLa- und HBE1-Zellen.

### Beispiel 6

### Gentransfer in Atemwegsepithelzellen unter Verwendung von binären und ternären Adenoviruskomplexen

Für diese Versuche wurde die Ratte Sigmodon hispidus ("Cotton Rat") verwendet, von der sich gezeigt hat, daß sie ein geeignetes Tiermodell für humane adenovirale Lungenerkrankungen darstellt (Pacini et al., 1984). Weiters wurden die im vorangegangenen Beispiel beschriebenen binären und ternären Komplexe verwendet.

### a) Transfektion von primären Atemwegs-Epithelzellkulturen

Die primären Kulturen wurden nach bekannten Methoden (Van Scott et al., 1986) hergestellt. Die dissoziierten Zellen wurden geerntet, 3 x mit F12-7X-Medium gewaschen und bei einer Dichte von 5 x 10⁵ Zellen pro Schale in 3 cm Gewebekulturschalen plattiert. Die Zellen wurden in F12-7X-Medium gehalten und beim Erreichen von 50 bis 75 % Konfluenz für die Gentransferversuche verwendet, was für gewöhnlich zwei bis drei Tage dauerte. Für die Gentransferversuche wurden die Komplexe direkt auf die Zellen aufgebracht und 24 h inkubiert. Für diese Versuche wurde pCMV-DNA verwendet. Das Plasmid pCMV wurde hergestellt, indem das BamHI-Insert des Plasmids pSTCX556 (Severne et al., 1988) entfernt, das Plasmid mit Klenow-Fragment behandelt und das HindIII/SspI sowie Klenow-behandelte Fragment aus dem Plasmid pRSVL, das die für Luciferase kodierende Sequenz enthält, bzw. die für β-Galaktosidase (MacGregor und Caskey, 1989) eingesetzt wurde, die erhaltenen Plasmide wurden pCMVL und pCMVβ-gal bezeichnet. Die Komplexbildung wurde analog pRSVL vorgenommen.

### i) Relative Höhe des Nettogentransfers

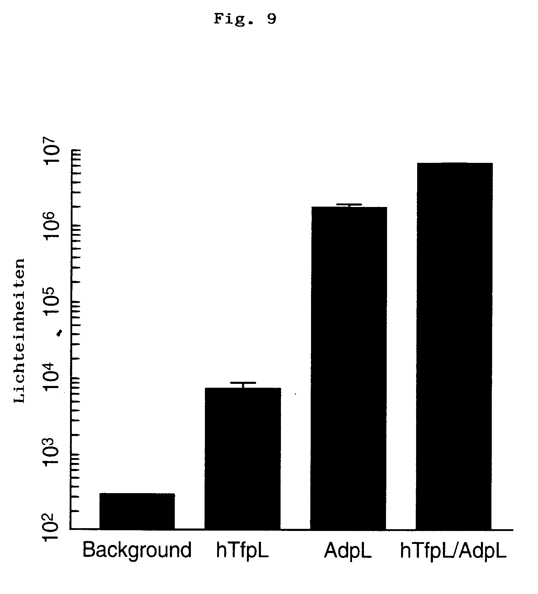

Für diese Versuche wurde das Reporterplasmid pCMVL verwendet. Die Zellen wurden nach 24 h auf Luciferasegenexpression untersucht; die Ergebnisse sind in Fig. 9 dargestellt. Der Untergrund zeigt die Messung der nicht-modifizierten Zellen an/die Ordinate stellt die Luciferasegenexpression als Lichteinheiten pro 25 µg Gesamtprotein, erhalten von Zellysaten, dar. Die Experimente wurden je 3 - 4 mal durchgeführt, die Werte sind Mittelwerte ± SEM.

### ii) Relative Transduktionshäufigkeit

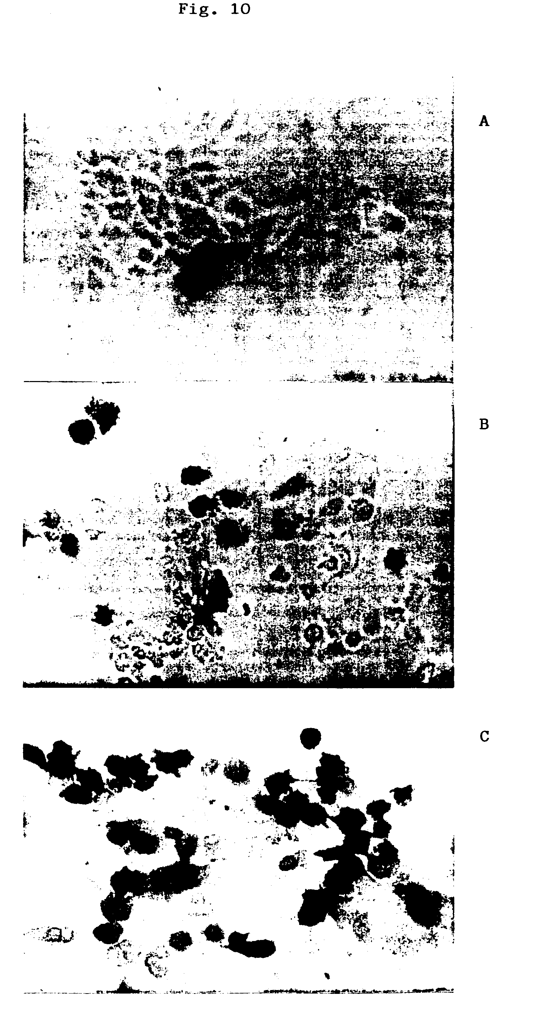

In diesen Versuchen wurde als Reporter-DNA das Plasmid pCMVβ-gal verwendet. Die Zellen wurden wie oben beschrieben transfiziert und nach 24 h die Reportergenexpression durch Färbung nach der von MacGregor et al., 1989, beschriebenen Methode bestimmt. Die Ergebnisse sind in Fig. 10 dargestellt (Vergrößerung: 320 x). A: hTfpL, B: AdpL, C: hTfpL/AdpL.

### b) Gentransfer über die intratracheale Route in vivo

Die Tiere wurden mit Methoxyfluran anästhesiert. Nach einem Vertikalschnitt in die ventrale Seite des Halses wurde die Luftröhre stumpf präpariert. Die Komplexe (250 bis 300 µl; 3 µg Plasmid-DNA) wurden unter Sicht direkt in die Luftröhre der in einem Winkel von 45° schräggelegten Tiere injiziert. Die Tiere wurden durch CO₂ getötet und die Luftröhre und die Lunge en bloc nach in situ Spülung mit kalter Phosphat-gepufferter Kochsalzlösung (PBS) geerntet. Für den Luciferasetest wurde das Lungengewebe in Extraktionspuffer homogenisiert, die Lysate auf Gesamtproteingehalt standardisiert und die Luciferasegenexpression gemessen wie beschrieben.

### i) Relative Höhe des Netto-Gentransfers in vivo

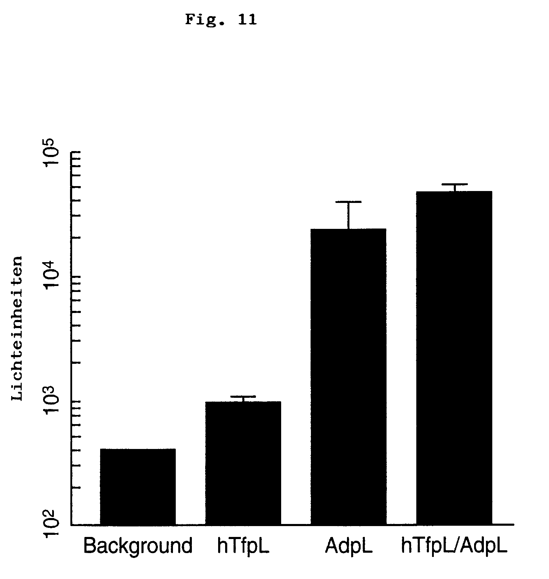

24 h nach der Transfektion wurde die Luciferaseexpression gemessen. Das Ergebnis ist in Fig. 11 dargestellt. Die angegebenen Lichteinheiten beziehen sich auf 1.250 µg Gesamtprotein, erhalten aus den Lungenlysaten. Die Experimente wurden je 3 - 4 x durchgeführt, die Ergebnisse sind als Mittelwerte ± SEM dargestellt.

### ii) Lokalisierung der heterologen Genexpression im Atemwegsepithel

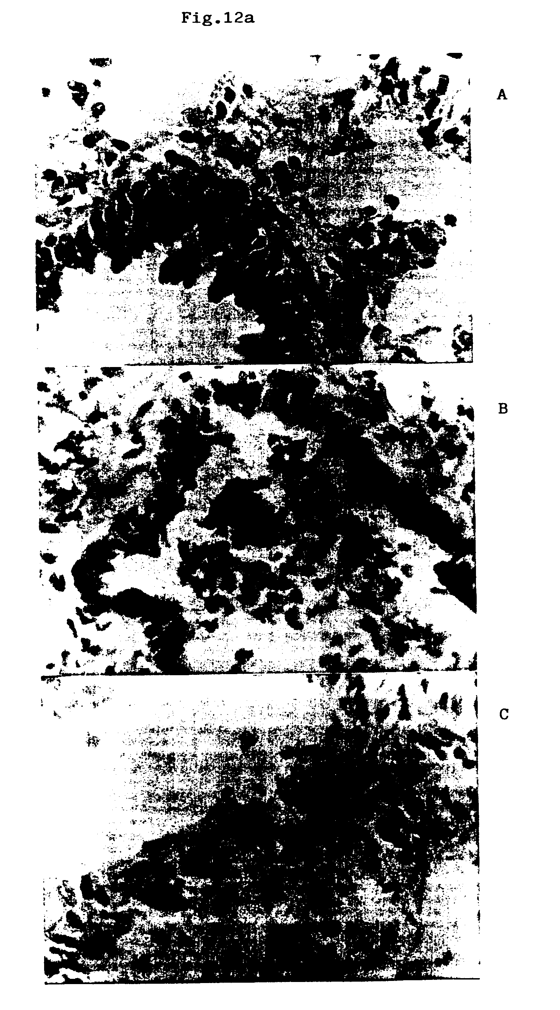

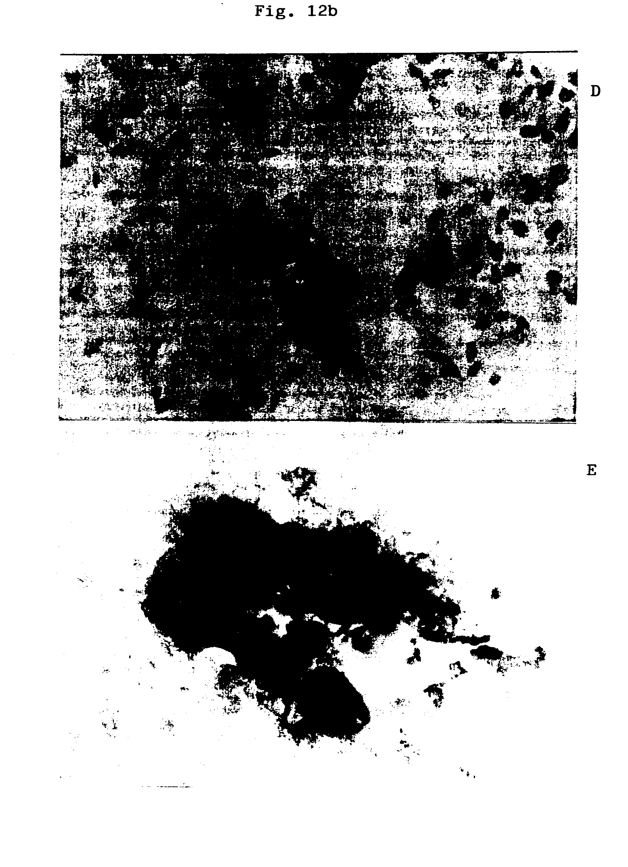

Für diese Versuche wurde als Reporter-DNA das Plasmid pCMVβ-gal verwendet; es wurden hTfpL/AdpL-Kombinations-Komplexe eingesetzt. 24 h nach der Injektion wurden 14 µm dicke Gefrierschnitte der geernteten Lunge auf Expression des Reportergens mittels Färbung mit X-gal und Gegenfärbung mit "Nuclear Fast Red" untersucht. Die Färbungen sind in Fig. 12 dargestellt (Vergrößerung: 600 x); sie zeigen die Ergebnisse der Transfektion von Ratten, die mit hTfpL/AdpL-Komplexen, enthaltend ein irrelevantes nicht-lacZ-Plasmid der Bezeichnung pRc/RSV oder pCMVβ-gal, enthaltend das lacZ-Reporterplasmid, behandelt worden waren. A: Beispiel eines Bronchiolus, behandelt mit Komplexen, enthaltend pRc/RSV; B: Beispiel eines Bronchus, behandelt mit Komplexen, enthaltend pCMVβ-gal; C: Beispiel der distalen Atemwegsregion, behandelt mit Komplexen, enthaltend pRc/RSV; D: Beispiel der distalen Atemwegsregion, behandelt mit Komplexen, enthaltend pCMVβ-gal; E: Vergrößerung der β-Galaktosidase-positiven Region aus Lungen, behandelt mit Komplexen, enthaltend pCMVβ-gal (Vergrößerung: 1.000 x).

### Beispiel 7

### Konstruktion von Konjugaten mit Spezifität für Lungenregionen

Es wurden Konjugate im Hinblick auf die selektive Bindung an den bewimperten Teil des Atemwegsepithels konstruiert. Die Konstruktion solcher Konjugate erfordert (im allgemeinen wie auch im speziellen Anwendungsfall) u.a. die Bestätigung der Bindungseigenschaften der Liganden-Kandidaten in der Konjugat-Konformation. Als Kandidat wurde das SNA Lectin gewählt.

### a) Herstellung von ternären Adenovirus-Polylysin/Lectin-Polylysin/DNA-Komplexen

Wie in den vorangegangenen Beispielen wurde das chimäre Adenovirus P202 (2 x 10¹⁰ Partikel) mit dem Antikörper-Polylysin-Konjugat MP301pL (1.25 µg) in 250 µl HBS vereinigt und 30 min bei Raumtemperatur inkubiert. Darauf wurde das Reporter-Plasmid pCMVL (6 µg in 125 µl HBS) zugegeben und weitere 30 min bei Raumtemperatur bebrütet. Ein im Handel erhältliches biotinyliertes Lectin SNA (E-Y Lab, San Mateo, CA; 2.8 µg) in 62.5 µl HBS wurde mit Streptavidin-Polylysin (1.35 µg in 62.5 µl HBS) vereinigt und zwecks Bildung von SNA-Polylysin 30 min bei Raumtemperatur stehen gelassen. Das SNA-Polylysin wurde mit obiger Reaktionsmischung zur Bildung von SNA-Adenovirus-Polylysin-DNA-Komplexen vereinigt. Zum Vergleich wurden Komplexe ohne den SNA-Liganden, wie in den vorangegangenen Beispielen beschrieben, hergestellt.

### b) In vivo Anwendung von Lectin-Komplexen in der Lunge

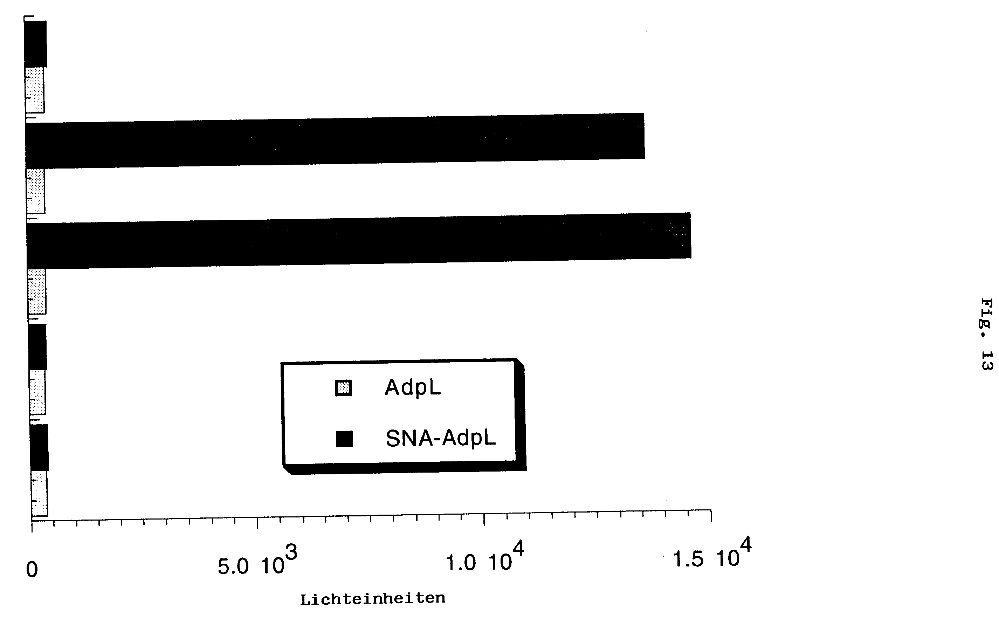

Dem zellspezifischen Tropismus des Lecins SNA für das bewimperte humane Atemwegsepithel wird im Frettchen entsprochen, das in den Experimenten als Tiermodell herangezogen wurde. Es wurden männliche Tiere mit ca. 1.5 kg Körpergewicht verwendet. Für jedes Tier wurden die in a) hergestellten Komplexe in vierfacher Menge verwendet. Die Tiere wurden anästhesiert und die Komplexe mittels Bronchoskop in den rechten mittleren Lungenlappen eingebracht. Nach 24 h wurden die verschiedenen Lungenregionen geerntet, homogenisiert und auf Luciferaseaktivität untersucht. Die untersuchten Lungenregionen umfaßten Teile, die mit den Komplexen bei deren Verabreichung nicht in Kontakt waren (linker oberer Lungenanteil, Parenchym des linken oberen Lappens, unterer Anteil der Luftröhre) und Teile, die mit den Komplexen in Kontakt waren (rechter mittlerer Lungenlappen, Parenchym des rechten mittleren Lungenlappens). Wie aus Fig. 13 ersichtlich, zeigten die Regionen, die mit den Komplexen in Kontakt waren, Luciferaseexpression (rechter mittlerer Lungenlappen, Parenchym des rechten mittleren Lungenlappens; 2. und 3. Balken), während die anderen Regionen (unterer Anteil der Luftröhre, 1.Balken; Parenchym des linken oberen Lappens, 4. Balken; linker oberer Lungenanteil, 5. Balken) keine Expression zeigten.

### c) Untersuchung der Spezifität des Liganden

Parallel dazu wurde die Spezifität der Bindung von Lectin-Konjugaten untersucht. Da kein anti-Lectin-Antikörper verfügbar war, wurden Transferrin/Lectin-Polylysin/DNA-Komplexe hergestellt und die Bindung mit einem primären anti-Transferrin-Antikörper, der durch einen sekundären Meerrettich-Peroxidase-gekoppelten anti-Maus-Antikörper verstärkt wurde. Es wurden die Konjugate im apikalen Teil der bewimperten Zellpopulation nachgewiesen, die Testanordung ließ jedoch keinen eindeutigen Schluß auf eine spezifische Bindung zu.

### Literatur:

Anderson, W.F., 1984, Science 226, 401-409.
Anderson, P. et al., 1982, J. Biol. Chem., 257, 11301-11304.
Abrahamson, D.R. et al., 1981, J. Cell Biol., 91, 270-280.
Asada-Kubota, M. et al., 1983, Exp. Pathol., 23, 95-101.
Ascoli, M. et al., 1978, J. Biol. Chem., 253, 7832-7838.
Ashwell, G. et al., 1982, Annu. Rev. Biochem., 51, 531-554.
Berkner, K.L. und Sharp, P.A., 1983, Nucl. Acids Res. 11, 6003-6020.
Berkner, K.L., 1988, BioTechniques 6, 616-629
Berns, K.I., 1990, Virology, 2nd Edition, Ed. by Fields, B.N., Knipe, D.M. et al., Raven Press Ltd., New York, 1743-1759.
Carpenter, G., 1984, Cell, 37, 357-358.
Cheng, S-Y. et al., 1980, Proc. Natl. Acad. Sci. USA, 77, 3425-3429.
Cotten, M., Laengle-Rouault, F., Kirlappos., H., Wagner, E., Mechtler, K., Zenke, M., Beug, H., und Birnstiel, M.L., 1990, Proc.Natl.Acad.Sci. USA 87, 4033-4037.
Ciliberto, G. et al., Cell, 1985, 41, 531-540.
Darnell, J., Lodish, H. und Baltimore, D., 1975, Mol. Cell Biol., Ed. Darnell, J., Freeman, New York, 567.
Davis, B.D. und Dulbecco R., 1980, Microbiology, 3. Edition, Ed. Davis, B.D. et al., Harper & Row, Sterilization and Disinfection, 1264-1274.
Defer, C., Belin, M., Caillet-Boudin, M. und Boulanger, P., 1990, J. Virol. 64, 3661-3673.
De Wet, J., Wood, K., DeLuca, M., Helinski, D. und Subramani, S., 1987, Mol. Cell. Biol. 7, 725-737.
Dulbecco, R., 1980, Microbiology, 3. Edition, Ed. Davis, B.D. *et al.*, Harper & Row, The Nature of Viruses, 853-884.
Eglitis und Anderson, 1988, Biotechniques, 6, 608-614.
Felgner, P.L., Gadek, T.R., Holm, M., Roman, R., Chan, H., Wenz, M., Northrop, J.P., Ringold, G.M. und Danielsen, M., 1987, Proc.Natl.Acad.Sci. USA 84, 7413-7417.
Felgner, P.L., Holm, M. und Chan, H., 1989, Proc.West.Pharmacol. Soc. 32, 115.
Fields, B.N. und Knipe, D.M., 1990, Virology 2nd edition, Raven Press Ltd., New York.
Ginsberg, H.S., 1980, Microbiology, 3. Edition, Ed. Davis, B.D. *et al.*, Harper & Row, Picornaviruses, 1095-1117.
Geysen *et al.*, 1984, Proc. Natl. Acad. Sci. USA, 81, 3998.
Geysen *et al.*, 1985, Proc. Natl. Acad. Sci. USA, 82, 178.
Geysen *et al.*, 1986, Mol. Immunol., 23, 709.
Geysen *et al.*, 1987, J. Immunol. Meth., 102, 259.
Goldstein, J.L. et al., 1982, Clin. Res., 30, 417-426.
Goldstein, J.L. et al., 1979, Proc. Natl Acad. Sci. USA, 76, 333-337.
Green, M. et al., 1989, Cell 58, 215-223.
Hearst, J. E. und Thiry, L., 1977, Nucl. Acids Res. 4, 1339-1347.
Heldin, C-H. et al., 1982, J. Biol. Chem., 257, 4216-4221.
Herskowitz, I., 1987, Nature 329, 219.
Hizuka, N. et al., 1981, J. Biol. Chem., 256, 4591-4597.
Holland, J.J., 1990, Virology, 2. Edition, Ed. Fields, B.N., Knipe, D.M. *et al*., Raven Press Ltd., New York, Defective Viral Genomes, 151-165.
Horwitz, M.S., 1990, Virology, 2. Edition, Ed. Fields, B.N., Knipe, D.M. *et al*., Raven Press Ltd., New York, Adenoviridae and their replication, 1679-1721.
Hosang, M. et al., 1987, EMBO J., 6, 1197-1202.
Huang, A.S., 1987, The Molecular Basis of Viral Replication, Hsg. Bercoff, R.P., Plenum Press New York and London, The Role of Defective Interfering (DI) Particles in Viral Infection, 191-194.
Huebers, H. und Finch, C., 1987, Physiol. Rev. 67, 520-582.
Hu, P.C., Collier, A.M. und Baseman, J.B., 1977, The Journal of Exp. Medicine 145, 1328-1343.
Imamura, K. et al., 1987, J. Immunol., 139, 2989-2992.
Inamine, J.M., Loechel, S. und Hu, P.C., 1988, Gene 73,175-183.
Kaplan, J. et al., 1979, J. Biol. Chem., 254, 7323-7328.
Klausner, R.D. et al., 1983, J. Biol. Chem., 258, 4715-4724.
Klausner, R.D. et al., 1983, Proc. Natl. Acad. Sci. USA 80, 2263-2266.
Kuhn, L.C. et al., 1982, Trends Biochem. Sci., 7, 299-302.
Kunkel, T.A., 1985, Proc.Natl.Acad.Sci. USA 82, 488-492.
Kurachi, K. et al., 1982, Proc. Natl. Acad. Sci. USA 1982, 79, 6461-6464.
Ledley, F.D. et al., 1989, In Biotechnology, Vol. 7b H-J (Rehm and G. Reed, eds. (VCH Verlagsgesellschaft, Weinheim) pp. 401-457.
Loyter, A., Scangos, G.A. und Ruddle, F.H., 1982, Proc.Natl. Acad.Sci. USA 79, 422.
MacGregor, G.R. und Caskey, C.T., 1989, Nucleic Acids Res. 17, 2365.
Malim, M. et al., 1989, Cell 58, 205-214.
Malone, R.W., Felgner, P.L. und Verma, I.M., 1989, Proc.Natl. Acad.Sci. USA 86, 6077.
Maniatis, T., Fritsch, E.F. und Sambrook, J. (1982) Molecular Cloning A Laboratory Manual. Cold Spring Harbor Laboratory, 474.
Marshall, S., 1985, J Biol. Chem., 250, 4133-4144.
Massague, J. et al., 1986, J. Cell. Physiol., 128, 216-222.
Maurer, R.A., 1989, Focus 11, 25.
McClure, M.O., Sommerfelt, M.A., Marsh, M. und Weiss, R.A., 1990, J. General Virol. 71, 767-773.
Mellman, I.S. et al., 1984, J. Cell Biol., 98, 1170-1177.
Mizel, S.B. et al., 1987, 1987, J. Immunol., 138, 2906-2912.
Pacini, D.L. et al., 1984, J. Infect. Dis. 150, 92-97.
Pastan, I., Seth, P., FitzGerald, D. und Willingham, M., 1986, in Virus attachment and entry into cells, Eds. Crowell, R.L. and Lonberg-Holm, K., (Am.Soc.for Microbiol., Washington DC) 141-146.
Philipson, L., Lonberg-Holm, K. und Pettersson, U., 1968, J. Virol. 2, 1064-1075.
Philipson, L., 1983, Curr. Top. Microbiol. Immunol. 109, 2.
Ponder, K.P. et al., 1991, Proc. Natl. Acad. Sci USA, 88, 1217-1221.
Posner, B.I. et al., 1982, J. Cell Biol., 93, 560-567.
Precious, B. und Russell, W.C., 1985, Virology: A practical approach, B.W.J. Mahy, ed. (IRL Press, Oxford, Washington, DC) pp. 193-205.
Roberts, M.M. et al., 1986, Science 232, 1148-1151.
Russell, W.C. et al., 1981, J. Gen. Virol., 56, 393-408.
Riordan, J.R. et al., 1989, Science, 245, 1066-1073.
Schalch, D.S. et al., 1986, Endocrinology, 118, 1590-1597.
Sennett, C. et al., 1981, Annu. Rev. Biochem., 50, 1053-1086.
Seth *et al.*, *Mol. Cell. Biol. 4*:1528-1533 (1984).
Sly, W. et al., 1982, J. Cell Biochem., 18, 67-85.
Smith, K.A. et al., 1985, Proc. Natl. Acad. Sci. USA, 82, 864-867.
Stahl, P.D. et al., 1978, Proc. Natl. Acad. Sci. USA, 75, 1399-1403.
Sullenger, B.A. et al., 1990, Cell 63, 601-608.
Svensson, U., 1985, J.Virol., 55, 442-449.
Trono, D. et al., 1989, Cell 59, 113-120.
Uchida, Y., Tsukada, U. und Sugimori, T., 1977, J. Biochem. 82, 1425-1433.
Valerio, D. et al., 1984, Gene, 31, 147-153.
Van Scott, M.R. et al., 1986, Exp. Lung Res. 11, 75-94.
Wagner, E., Zenke, M., Cotten, M., Beug, H. und Birnstiel, M.L., 1990, Proc.Natl.Acad.Sci. USA 87, 3410-3414.
Wagner, E., Cotten, M., Foisner, R. und Birnstiel, M.L., 1991a, Proc.Natl.Acad.Sci. USA 88, 4255-4259.
Wagner, E., Cotten, M., Mechtler, K., Kirlappos, H. und Birnstiel, M.L., 1991b, Bioconjugate Chemistry 2, 226-231.
Walker, F. et al., 1987, J. Cell Physiol., 130, 255-261.
Watson, J.D. et al., 1987, Mol. Biol. of the Gene, Benjamin/Cummings Publ. Company Inc., 676-677.
Willumsen, J.J., Davis, C.W. und Boucher, R.C., 1989, Am. J. Physiol. 256, C1033-C1044.
Wood, W.I. et al., 1984, Nature, 312, 330-337.
Wu, G.Y. und Wu, C.H., 1987, J. Biol. Chem. 262, 4429-4432.
Zamecnik, P.C., Goodchild, J., Taguchi, Y. und Sarin, P.S., 1986, Proc.Natl.Acad.Sci. USA 83, 4143-4146.
Zenke, M., Steinlein, P., Wagner, E., Cotten, M., Beug, H. und Birnstiel, M.L., 1990, Proc.Natl.Acad.Sci. USA 87, 3655-3659.
Remington's Pharmaceutical Sciences, 1980, Mack Publ. Co., Easton, PA, Osol (ed.).

## Patentansprüche

1. Neues Konjugat, das die Fähigkeit besitzt, mit Nukleinsäure Komplexe zu bilden und das aus einem Internalisierungsfaktor und einer Nukleinsäureaffinen Substanz besteht, zum Einführen von Nukleinsäure in höhere einkaryotische Zellen, dadurch gekennzeichnet, daß der Internalisierungsfaktor ein Virus ist, das an die Nukleinsäure-affine Substanz über einen Antikörper derart gebunden ist, daß es die Fähigkeit besitzt, als Bestandteil des Konjugat/Ninkleinsäure-Komplexes in die Zelle einzudringen und den Inhalt der Endosomen, in denen der Komplex nach Eintritt in die Zelle lokalisiert ist, ins Zytoplasma freizusetzen.

2. Konjugat nach Anspruch 1, dadurch gekennzeichnet, daß der Antikörper an ein Virusprotein bindet, das an der Funktion des Virus, in die Zelle einzudringen und den Endosomeninhalt freizusetzen, nicht beteiligt ist.

3. Konjugat nach Anspruch 1 oder 2, dadurch gekennzeichnet, daß das Virus ein Adenovirus ist.

4. Konjugat nach Anspruch 3, dadurch gekennzeichnet, daß der Antikörper an ein Epitop in der Hexonregion bindet.

5. Konjugat nach den Ansprüchen 3 und 4, dadurch gekennzeichnet, daß das Adenovirins ein chimäres Virus ist, das in der für das Hexonprotein kodierenden Sequenz anstelle der Codons 188 - 194 die für die Aminosäuren 914 bis 928 des Mycoplasma pneumoniae Proteins P1 kodierende Sequenz enthält und daß der Antikörper an die P1-Region bindet.

6. Konjugat nach einem der Ansprüche 2 bis 5, dadurch gekennzeichnet, daß der Antikörper ein monoklonaler Antikörper ist.

7. Konjugat nach einem der Ansprüche 1 bis 6, dadurch gekennzeichnet, daß die Nukleinsäure-affine Substanz ein Polykation ist.

8. Konjugat nach Anspruch 7, dadurch gekennzeichnet, daß das Polykation Polylysin ist.

9. Komplex aus Nukleinsäure und einem Konjugat aus Internalisierungsfaktor und Nukleinsäure-affiner Substanz, dadurch gekennzeichnet, daß er als Konjugat eines der in den Ansprüchen 1 bis 8 definierten Konjugate enthält.

10. Komplex nach Anspruch 9, dadurch gekennzeichnet, daß die Nukleinsäure ein Genkonstrinkt ist, das ein therapeutisch wirksames Gen enthält.

11. Komplex nach Anspruch 10, dadurch gekennzeichnet, daß das Gen ein gentherapeutisch wirksames Gen bzw. ein Genabschnitt ist.

12. Komplex nach Anspruch 10, dadurch gekennzeichnet, daß das Genkonstrukt einen Abschnitt enthält, von dem Zellfunktionen spezifisch inhibierende RNA-Moleküle transkribierbar sind.

13. Komplex nach Anspruch 9, dadurch gekennzeichnet, daß das Virus per se ein Internalisierungsfaktor für die Zelle ist.

14. Komplex nach Anspruch 9, dadurch gekennzeichnet, daß das Konjugat ein Virus enthält, daß nicht per se ein Internalisierungsfaktor für die Zelle ist und daß der Komplex außerdem einen Internalisierungsfaktor enthält.

15. Komplex nach Anspruch 9 oder 14, dadurch gekennzeichnet, daß er ein weiteres Konjugat aus Nukleinsäure-affiner Substanz und einem Internalisierungsfaktor, der spezifisch für einen Oberflächenrezeptor der höheren eukaryotischen Zelle ist, enthält, wobei das Virus-Konjugat und das Internalisierungsfaktor-Konjugat mit der Nukleinsäure komplexiert sind.

16. Komplex nach Anspruch 15, dadurch gekennzeichnet, daß die Nukleinsäure-affine Substanz des zweiten Konjugats ein organisches Polykation ist.

17. Komplex nach Anspruch 16, das das Polykation Polylysin ist.

18. Komplex nach Anspruch 15, dadurch gekennzeichnet, daß der zweite Internalisierungsfaktor Transferrin ist.

19. Komplex nach Anspruch 15, dadurch gekennzeichnet, daß der zweite Internalisierungsfaktor ein Lectin ist.

20. Verfahren zum Einbringen von Nukleinsäure in höhere eukaryotische Zellen, dadurch gekennzeichnet, daß die Zellen mit einem der in den Ansprüchen 9 bis 19 definierten Komplexe behandelt werden.

21. Pharmazeutische Zubereitung, dadurch gekennzeichnet, daß sie als wirksame Komponente einen der in den Ansprüchen 9 bis 19 definierten Komplexe enthält.

22. Transfektionskit, enthaltend eine Trägereinheit, in der sich zwei oder mehrere Behälter befinden, wobei ein erster Behälter eine Nukleinsäure-affine Substanz, gebunden an einen Antikörper, und ein zweiter Behälter ein Virus, mit dem der Antikörper immunreaktiv ist, enthält, wobei das Virus die Fähigkeit besitzt, in eine höhere eukaryotische Zelle einzudringen, wenn es Teil eines Komplexes zwischen der Nukleinsäure-affinen Substanz und einer Nukleinsäure ist und wobei das Virus die Fähigkeit hat, den Inhalt der Endosomen, in denen der Komplex nach Eintritt in die Zelle lokalisiert ist, ins Zytoplasma freizusetzen.

23. Transfektionskit, enthaltend eine Trägereinheit, in der sich ein oder mehrere Behälter befinden, wobei ein erster Behälter eine Nukleinsäure-affine Substanz enthält, die immunologisch über einen Antikörper an ein Virus gebunden ist, das die Fähigkeit hat, in eine höhere eukaryotische Zelle einzudringen, wenn es Teil eines Komplexes zwischen der Nukleinsäure-affinen Substanz und einer Nukleinsäure ist und wobei das Virus die Fähigkeit hat, den Inhalt der Endosomen, in denen der Komplex nach Eintritt in die Zelle lokalisiert ist ins Zytoplasma freizusetzen.

24. Transfektionskit nach Anspruch 22 oder 23, dadurch gekennzeichnet, daß außerdem einer der Behälter ein zweites Konjugat aus einem Internalisierungsfaktor für eine höhere eukaryotische Zelle und einer Nukleinsäure-affinen Substanz enthält.
